(19) Europäisches Patentamt
European Patent Office
Office européen des brevets

(11) EP 2 495 563 A1

(12) **EUROPEAN PATENT APPLICATION**
published in accordance with Art. 153(4) EPC

(43) Date of publication:
**05.09.2012 Bulletin 2012/36**

(21) Application number: **10826654.5**

(22) Date of filing: **25.10.2010**

(51) Int Cl.:
***G01N 33/50*** *(2006.01)* ***A61K 49/00*** *(2006.01)*
***G01N 33/15*** *(2006.01)*

(86) International application number:
**PCT/JP2010/068812**

(87) International publication number:
**WO 2011/052522 (05.05.2011 Gazette 2011/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2009 JP 2009245330**

(71) Applicant: **Fujifilm RI Pharma Co., Ltd.**
**Tokyo 104-0031 (JP)**

(72) Inventors:
- **SAKURAI, Kazuhisa**
  **Sammu-shi**
  **Chiba 289-1592 (JP)**
- **NAGANO, Akio**
  **Sammu-shi**
  **Chiba 289-1592 (JP)**

(74) Representative: **Blodig, Wolfgang et al**
**Wächtershäuser & Hartz**
**Patentanwaltspartnerschaft**
**Ottostrasse 4**
**80333 München (DE)**

# (54) DIAGNOSTIC AGENT FOR INFECTIOUS DISEASES

(57) A diagnostic agent for infectious diseases which is capable of distinguishing among different kinds of bacterial species and which allows simple and non-invasive measurement and/or imaging in a short period of time is provided; and a screening method for a therapeutic agent for infectious diseases caused by microorganisms are provided.

A diagnostic agent for infectious diseases caused by nitroimidazole susceptible microorganisms, containing an imidazole derivative or a fused imidazole derivative having at least one nitro group on an imidazole ring, or a labeled form thereof as an active ingredient is provided.



Printed by Jouve, 75001 PARIS (FR)

## Description

[Technical Field]

The present invention relates to a diagnostic agent for infectious diseases capable of detecting nitroimidazole susceptible microorganisms.

[Background Art]

Chemotherapy for infectious diseases basically involves determining the causative microorganisms and immediately starting treatment by administering an adequate dose of antimicrobial drugs to which the microorganisms are susceptible for a short period of time (Non Patent Literature 1).

Normally, in order to determine the causative microorganisms, a culture test is performed. Currently, however, determination of the causative microorganisms may sometimes be difficult, for example, in the case of microorganisms having low detection sensitivity to a culture test. Also, when indigenous bacteria are detected by culture, it is difficult to judge whether those indigenous bacteria are involved in the disease or contaminant (inadvertent incorporation). Also, technically, the microbial culture test has problems such that it is time consuming. While varying depending on the kind of microorganism, the microbial culture test requires several days to several weeks. Moreover, because further time is needed to obtain the result of a drug sensitivity test of the microorganisms which are isolated and then subsequently cultured, immediate beginning of appropriate treatment is difficult. Also, when the deep part of the living body is infected, it may be difficult to carry out a culture test because collection of a specimen for culture involves invasive procedure.

Due to the diagnostic method-relating problems as stated above, treatment of infectious diseases tends to rely on a physician's experience, which causes intractability of treatment and aggravation of the disease. Moreover, even when drug-sensitive antimicrobial drugs are used, a sufficient therapeutic amount of the drug may not be delivered to the focus of infection, and also, there are problems of side effects such as microbial substitution. Therefore, an antimicrobial drug is necessarily used in an appropriate amount for a short period of time.

Based on the foregoing, for treatment of infectious diseases, a diagnostic method capable of measuring viable microorganisms non-invasively by a simple operation in a short period of time is demanded.

Diagnosis of infectious diseases caused by obligate anaerobes, among the infectious diseases, particularly requires a long time and involves extremely complicated operations (Non Patent Literature 2).

Diagnosis of infectious diseases caused by obligate anaerobes requires an anaerobic culture test. Because many of the bacterial species to be examined by this test die immediately or exhibit impaired growth upon exposure to air during specimen sampling, the rate of detection by isolation culture is low. Also, many of the infectious diseases caused by obligate anaerobes involve bacterial species composing the normal flora of humans. Thus, current diagnostic methods such as an isolation culture method result many false positives, making it difficult to judge whether the tested bacteria are involved as the causative bacteria.

To make matters more difficult, obligate anaerobes often cause mixed infections with aerobic bacteria such as facultative anaerobes, or multiple species of obligate anaerobes often cause simultaneous infections. Since facultative anaerobes grow well in anaerobic culture as well, confirmation of the presence of obligate anaerobes is necessary, and even a skilled technician would need one week or longer to do this. Also, test results often vary due to a difference in the technical skill of the laboratory technicians.

Also, when the deep part is infected, specimen sampling involves invasive procedure, which is not only painful to patients and accompanies a problem of a risk of spreading the area of infection.

It is reported that, among the patients infected with obligate anaerobes from whom obligate anaerobes have been detected by blood culture, the mortality rate for the group of patients who have been appropriately treated for obligate anaerobes is 17%, whereas the mortality rate for the group of patients who have not been appropriately treated for obligate anaerobes is 55%, indicating the importance of diagnosis and treatment of obligate anaerobes (Non Patent Literature 3).

Meanwhile, conventionally, there are many reports relating to radioactive diagnostic imaging agents using radioisotope-labeled nitroimidazole derivatives (Patent Literature 1, Non Patent Literatures 4 and 5). A technique of imaging the location where cancer cells which have acquired hypoxia tolerance and have been surviving under a hypoxic environment are present in cancer patients using nitroimidazole derivatives is known (Non Patent Literature 6). Also, visualization of the hypoxic lesion site in patients with odontogenic infections by a positron emission tomographic image using a fluorine-18-labeled fluoromisonidazole ([$^{18}$F]MISO), which is a nitroimidazole derivative, has been reported (Non Patent Literature 7).

Also, nitroimidazole derivatives are known to have antimicrobial actions, and among them, several kinds of 5-nitroimidazoles are therapeutically used as antimicrobial drugs. Metronidazole, which is a 5-nitroimidazole derivative, is a representative antimicrobial drug that has been used worldwide for the treatment of infectious diseases since half a

century ago. Metronidazole has activities on all the obligate anaerobes, microaerophilic bacteria, for example Helicobacter pylori, certain kinds of protozoan parasites, for example Trichomonas, Entamoeba histolytica, and Giardia lamblia, and currently still remains as an excellent antimicrobial drug (Patent Literatures 2 to 6, Non Patent Literatures 8 to 10). Also, metronidazole is known to have characteristics such that it is resistant against facultative anaerobes even under an anaerobic environment, which facultative anaerobes can proliferate even under an anaerobic (hypoxic) environment, as well as obligate anaerobes (Non Patent Literature 11).

Further, recently, nitroimidazole derivatives have been found to have characteristics which are not found in the existing antituberculosis drugs such that they even act on tuberculosis in the resting stage of division (Non Patent Literatures 8 and 12), and development of a nitroimidazole derivative as a novel antituberculosis drug is currently ongoing (Patent Literatures 2, 7, and 8, Non Patent Literatures 13 and 14).

As described above, nitroimidazole derivatives not only have characteristics of accumulating in the hypoxic lesion site but also have an aspect as an antimicrobial drug effective for the treatment of infectious diseases. In this regard, nitroimidazole derivatives are known to have characteristics such that their efficacy varies depending on the pathogenic microorganisms even under a hypoxic environment, and thus have selective sensitivity.

[Prior Art Literature]

[Patent Literature]

[Patent Literature 1] JP-A-2003-509413
[Patent Literature 2] JP-A-2009-521464
[Patent Literature 3] JP-A-57-181066
[Patent Literature 4] JP-B-4-56031
[Patent Literature 5] JP-A-11-508270
[Patent Literature 6] JP-A-9-143071
[Patent Literature 7] JP-A-2005-330266
[Patent Literature 8] JP-A-2004-149527

[Non Patent Literature]

[Non Patent Literature 1] Kokinyaku shiyo no guideline (literally translated as "guideline for application of antimicrobial drugs"): The Japanese Association for Infectious Diseases, Japanese Society of Chemotherapy
[Non Patent Literature 2] Kenkiseikin kansensho shindan/chiryo guideline 2007 (literally translated as "diagnostic and therapeutic guidelines for infectious diseases caused by anaerobic bacteria") 2007: Japanese Society of Chemotherapy, Nihon kenkiseikin kansennsho kenkyukai (literally translated as Japanese study group for anaerobic bacterial infection)
[Non Patent Literature 3] Salonen JH, Clinical Infectious Diseases 26: 1413 to 1417 (1998)
[Non Patent Literature 4] Chu T, Bioorg Med Chem Lett. 9; 14 (3): 747 to 749 (2004)
[Non Patent Literature 5] Mukai T, Bioorg Med Chem. 1; 17 (13): 4285 to 4289 (2009)
[Non Patent Literature 6] Cherk MH, J Nucl Med. 47 (12) : 1921 to 1926 (2006)
[Non Patent Literature 7] Liu RS, Eur J Nucl Med. 23 (10): 1384 to 1387 (1996)
[Non Patent Literature 8] Samuelson J, Antimicrob Agents Chemother. 43 (7): 1533 to 1541 (1999)
[Non Patent Literature 9] Prince HN, Appl Microbiol. 18 (5): 728 to 730 (1969)
[Non Patent Literature 10] The Merck Manuals, the 18th edition, in Japanese
[Non Patent Literature 11] Cowan and Steel's Manual for the Identification of Medical Bacteria, the 3rd edition: 29
[Non Patent Literature 12] Sun Z, Tuber Lung Dis. 79 (5): 319 to 320 (1999)
[Non Patent Literature 13] Singh R, Science. 28; 322 (5906): 1392 to 1395 (2008)
[Non Patent Literature 14] Kim P, J Med Chem. 52: 1317 to 1328 (2009)

[Summary of Invention]

[Problem to be Solved by the Invention]

An object of the present invention is to provide a diagnostic agent for infectious diseases which is capable of

distinguishing among different kinds of bacterial species and which allows simple and non-invasive measurement and/or imaging in a short period of time. Another object of the present invention is to provide a screening method for a therapeutic agent for infectious diseases caused by microorganisms.

[Means for Solving the Problem]

In view of the foregoing, the present inventors conducted various studies in order to achieve the aforementioned objects. As a result, they have found that, unexpectedly, a nitroimidazole derivative has characteristics such that it selectively accumulates in a greater amount in nitroimidazole susceptible microorganisms rather than in nitroimidazole non-susceptible microorganisms even under an anaerobic (hypoxic) environment, and it does but accumulate in inflammation in which viable microorganisms are not involved, and thus is useful as a diagnostic agent for infectious diseases caused by nitroimidazole susceptible microorganisms. They have further found that a novel therapeutic agent for infectious diseases can be screened by utilizing the aforementioned accumulation property of the nitroimidazole derivatives in microorganisms. They completed the present invention based on these findings.

That is, the present invention provides a diagnostic agent for infectious diseases caused by nitroimidazole susceptible microorganisms, containing an imidazole derivative or a fused imidazole derivative having at least one nitro group on an imidazole ring, or a labeled form of the derivative as an active ingredient.

The present invention also provides an imidazole derivative or a fused imidazole derivative having at least one nitro group on an imidazole ring, or a labeled form of the derivative for use in the diagnosis of infectious diseases caused by nitroimidazole susceptible microorganisms.

The present invention also provides use of an imidazole derivative or a fused imidazole derivative having at least one nitro group on an imidazole ring, or a labeled form of the derivative for the production of a diagnostic agent for infectious diseases caused by nitroimidazole susceptible microorganisms.

The present invention also provides a diagnostic method for infectious diseases caused by nitroimidazole susceptible microorganisms, characterized by using an imidazole derivative or a fused imidazole derivative having at least one nitro group on an imidazole ring, or a labeled form of the derivative.

The present invention also provides a screening method for a therapeutic agent for infectious diseases caused by microorganisms, characterized by detecting binding of an imidazole derivative or a fused imidazole derivative having at least one nitro group on an imidazole ring, or a labeled form of the derivative to viable microorganisms in the presence of a test substance.

[Advantageous Effects of Invention]

By simply administering the diagnostic agent for infectious diseases of the present invention to patients with inflammation, an image of the site of infection with microorganisms susceptible to antimicrobial drugs having a nitroimidazole structure can be rapidly and non-invasively obtained, and simultaneously, the therapeutic effect can be predicted from the evaluation of the migration of the diagnostic agent to the site of infection. Further, because the amount of viable microorganisms can be non-invasively evaluated from the image thus obtained, the therapeutic effect and the time of completion of the antimicrobial drug therapy can be rapidly and objectively determined. Also, the screening method of the present invention is useful for screening a preventive and/or therapeutic agent for infectious diseases caused by nitroimidazole susceptible microorganisms.

[Brief Description of Drawings]

[Figure 1] Figure 1 shows the uptake of metronidazole by each microorganism under an anaerobic environment.
[Figure 2] Figure 2 shows the uptake of [$^{19}$F]MISO by each microorganism under an anaerobic environment.
[Figure 3] Figure 3 shows the uptake of Compound 1 by each microorganism under an anaerobic environment.
[Figure 4] Figure 4 shows the uptake of Compound 2 by each microorganism under an anaerobic environment.
[Figure 5] Figure 5 shows the uptake of [$^{18}$F]MISO by each microorganism under an anaerobic environment.
[Figure 6] Figure 6 shows the uptake of Compound 3 by each microorganism under an anaerobic environment.
[Figure 7] Figure 7 shows a relationship between the uptake amount of Compound 3 by Bacteroides fragilis under an anaerobic environment and the number of viable obligate anaerobes.
[Figure 8] Figure 8 shows accumulation of Compounds 1 and 2 in the rat model with infectious disease caused by each microorganism.
[Figure 9] Figure 9 shows the uptake of Compound 2 by each microorganism under an anaerobic environment.
[Figure 10] Figure 10 shows the uptake of [$^{19}$F]FMISO by each microorganism under an anaerobic environment.

[Modes for Carrying out the Invention]

The active ingredient of the diagnostic agent for infectious diseases of the present invention is an imidazole derivative or a fused imidazole derivative having at least one nitro group on an imidazole ring, or a labeled form of the derivative. Examples of this imidazole derivative or fused imidazole derivative include a compound represented by the following formula (1) or a labeled form thereof:

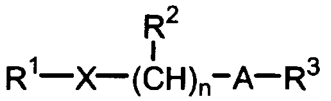 (1)

wherein, X represents an imidazole ring or a fused imidazole ring having at least one nitro group on an imidazole ring, $R^1$ represents a hydrogen atom or an alkyl group, n units of $R^2$ may be the same or different and each represent a hydrogen atom, a hydroxyl group, or an alkyl group, n represents a number of 0 to 5, A represents a single bond, an oxygen atom, a sulfur atom, $SO_2$ NH, CO, NHCO, or CONH, and $R^3$ represents a hydrogen atom, an optionally substituted alkyl group, an optionally substituted aryl group, an optionally substituted aralkyl group, or an optionally substituted heterocyclic group (refer to Patent Literatures 1 to 8, Non Patent Literatures 4 to 13).

In the formula (1), examples of X include the following structures (a) to (d):

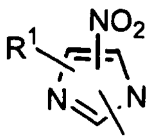
(a)

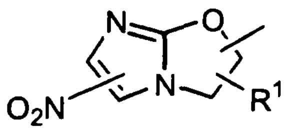
(b)

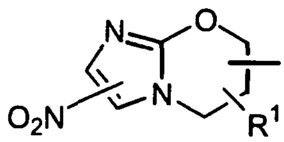
(c)

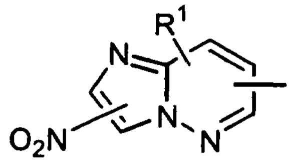
(d)

wherein, $R^1$ is the same as above.
Among the aforementioned structures (a) to (d), the structures (a), (b), and (c) are particularly preferred.

$R^1$ is a substituent on an imidazole ring or a fused imidazole ring, and is preferably a hydrogen atom or a $C_{1-5}$ alkyl group, more preferably a hydrogen atom, a methyl group, an ethyl group, a propyl group, an isopropyl group, and the like, and particularly preferably a hydrogen atom or a methyl group.

The n units of $R^2$ may be the same or different and each represent a hydrogen atom, a hydroxyl group, or an alkyl group; and, a hydrogen atom, a hydroxyl group, or a $C_{1-5}$ alkyl group is preferred. Here, examples of the alkyl group include a methyl group, an ethyl group, a propyl group, and an isopropyl group. The n represents a number of 0 to 5, more preferably a number of 0 or 1 to 3. Examples of $-(CH(R^2))_n-$ include a single bond, $-CH_2-$, $-CH_2CH_2-$, $-CH_2CH_2CH_2-$, $-CH(OH)-$, and $-CH(CH_3)-$.

The A represents a single bond, an oxygen atom, a sulfur atom, NH, $SO_2$, CO, NHCO, or CONH, and among these a single bond, an oxygen atom, NH, or NHCO is particularly preferred.

Examples of the optionally substituted alkyl group represented by $R^3$ include a $C_{1-6}$ alkyl group optionally having 1 to 3 substituents selected from a halogen atom, a hydroxyl group, an amino group, an alkanoyl group, an alkanoyloxy group, a cyano group, a nitro group, a carboxyl group, an optionally substituted alkoxy group, an optionally substituted aryl group, and an optionally substituted heterocyclic group. Here, examples of the $C_{1-6}$ alkyl group include a methyl group, an ethyl group, a propyl group, an isopropyl group, a butyl group, an isobutyl group, a sec-butyl group, a tert-butyl group, a pentyl group, an isopentyl group, and a hexyl group.

Examples of the optionally substituted aryl group represented by $R^3$ include a $C_{6-14}$ aryl group optionally having 1 to 3 substituents selected from a $C_{1-5}$ alkyl group, a halogen atom, hydroxyl group, an amino group, an alkanoyl group, an alkanoyloxy group, a cyano group, a nitro group, a carboxyl group, an optionally substituted alkoxy group, and an optionally substituted heterocyclic group. Here, examples of the $C_{6-14}$ aryl group include a phenyl group, a naphthyl group, and a phenanthryl group.

Examples of the optionally substituted aralkyl group represented by $R^3$ include a phenyl-$C_{1-5}$ alkyl group optionally having 1 to 3 substituents selected from a $C_{1-5}$ alkyl group, a hydroxyl group, an amino group, an alkanoyl group, an alkanoyloxy group, a cyano group, a nitro group, a carboxyl group, and an optionally substituted alkoxy group.

Example of the optionally substituted heterocyclic group represented by $R^3$ include a saturated or unsaturated

heterocyclic group optionally having 1 to 3 substituents selected from a $C_{1-5}$ alkyl group, a halogen atom, a hydroxyl group, an amino group, an alkanoyl group, an alkanoyloxy group, a cyano group, a nitro group, a carboxyl group, and an optionally substituted alkoxy group. Here, examples of the heterocyclic group include a pyrrolidinyl group, a piperidinyl group, a piperazinyl group, a pyrrole group, a pyridyl group, an oxazolyl group, a thiazolyl group, a pyridazinyl group, an imidazolyl group, a pyrazinyl group, and a morpholino group.

Among the compounds of the formula (1), more preferred embodiments include the following compounds:

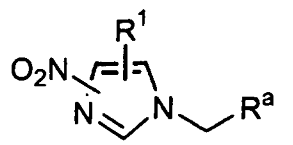

(a-1)

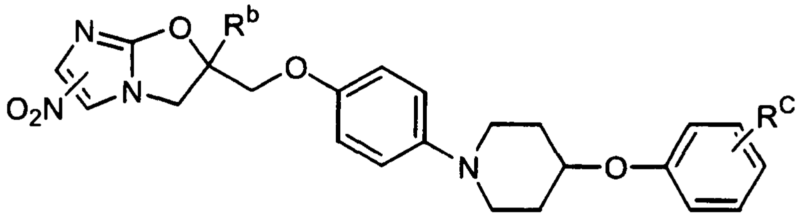

(b-1)

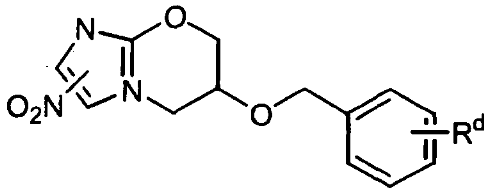

(c-1)

wherein, $R^1$ represents a hydrogen atom or a $C_{1-5}$ alkyl group, $R^a$ represents a $C_{1-5}$ alkyl group optionally having 1 to 3 substituents selected from a halogen atom, a hydroxyl group, and an alkanoyloxy group, a $C_{1-5}$ alkoxy group optionally having 1 to 3 substituents selected from a halogen atom, a hydroxyl group, and an alkanoyloxy group, or a benzoylaminomethyl group optionally having 1 to 3 substituents selected from a halogen atom, a hydroxyl group, and an alkanoyl group, $R^b$ represents a $C_{1-5}$ alkyl group, and $R^c$ and $R^d$ each represent a halogenoalkoxy group.

In the aforementioned formula, $R^1$ is particularly preferably a hydrogen atom or a methyl group. Examples of $R^a$ include a hydroxy-$C_{1-5}$ alkyl group, a halogeno-$C_{1-5}$ alkyl group, a halogeno-hydroxy-$C_{1-5}$ alkyl group, a hydroxy-$C_{1-5}$ alkoxy group, a halogeno-$C_{1-5}$ alkoxy group, a halogeno-hydroxy-$C_{1-5}$ alkoxy group, a halogeno-alkanoyloxy-$C_{1-5}$ alkoxy group, and a halogenobenzoylamino-$C_{1-5}$ alkyl group. Here, examples of the $C_{1-5}$ alkyl group include a methyl group, an ethyl group, a propyl group, and an isopropyl group. Examples of the $C_{1-5}$ alkoxy group include a methoxy group, an ethoxy group, and an isopropoxy group.

$R^c$ and $R^d$ are each a trihalogenomethoxy group, and particularly, a trifluoromethoxy group is particularly preferred.

More preferred examples of the imidazole derivative of the formula (1) include metronidazole, tinidazole, benznidazole, fluoromisonidazole, ornidazole, nimorazole, 1-[2-(2-methyl-5-nitro-1H-1-imidazolyl)ethyl]-3-iodobenzamide, 1-[2-(2-nitro-1H-1-imidazolyl)ethyl]-3-iodobenzamide, (S)-2-nitro-6-((4-trifluoromethoxy)-benzyloxy)-6, 7-dihydro-5H-imidazole[2,1-b][1,3] oxazine (Compound 4: Patent Literature 5), and (R)-2-methyl-6-nitro-2-((4-(4-(4-(trifluoromethoxy) phenoxy)-piperidin-1-yl)phenoxy)methyl)-2,3-dihydroimidazo[2,1-b]oxazole (Compound 5: Patent Literature 8).

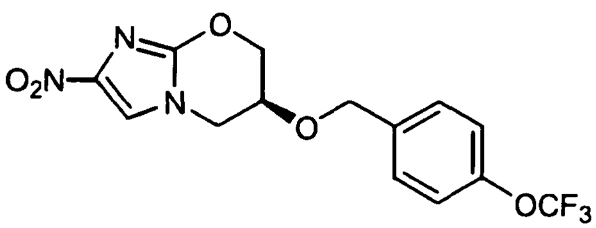

Compound 4

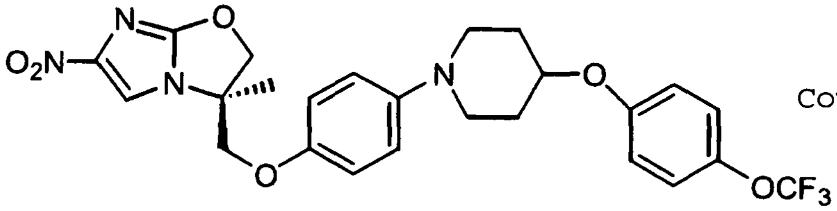

Compound 5

The labeled form of the above compound may be either a labeled form for in vitro diagnostic use or a labeled form for in vivo diagnostic use.

A label for in vitro diagnostic use may be a radionuclide such as $^{3}H$, $^{14}C$, $^{32}P$, $^{35}S$, and $^{125}I$, a fluorescent or chemiluminescent compound such as fluorescein, isothiocyanate, rhodamine, and luciferin, or an enzyme such as alkaline phosphatase, β-galactosidase, and horseradish peroxidase.

Also, for in vivo diagnostic use, the aforementioned nitroimidazole derivative of the formula (1) containing a detectable moiety useful for diagnostic imaging (hereinbelow, simply referred to as a nitroimidazole derivative) can be used. Examples of the detectable moiety include a radionuclide, a radiopaque atom, a near-infrared fluorescent compound, a paramagnetic compound, and an atom with nuclear spin. The nitroimidazole derivative is administered to a mammal preferably the blood stream, and the presence and location of the aforementioned nitroimidazole derivative is detected externally.

More specifically, a radioactive diagnostic agent for infectious diseases containing the aforementioned nitroimidazole derivative having, in its structure, a radionuclide, for example any one of $^{11}C$, $^{13}N$, $^{15}O$, $^{18}F$, $^{34}mCl$, $^{38}Cl$, $^{75}Br$, $^{76}Br$, $^{77}Br$, $^{80}mBr$, $^{80}Br$, $^{82}Br$, $^{121}I$, $^{123}I$, $^{124}I$, $^{126}I$, and $^{131}I$, an X-ray diagnostic agent for infectious diseases containing one or more X-ray absorbing atoms, i.e., an atom having an atomic number of 20 or more, a diagnostic agent for infectious diseases for nuclear magnetic resonance having one or more atoms with nuclear spin such as $^{19}F$ and $^{11}B$, a diagnostic agent for infectious diseases for electron paramagnetic resonance containing a paramagnetic compound such as nitroxide, a compound having fluorescence in the near-infrared region such as a near-infrared fluorescent compound containing a fluorophore such as BODIPY, or a diagnostic agent for infectious diseases enabling visualization of the location of the nitroimidazole derivative by similar methods are used.

Further, a nitoroimidazole derivative composed of a metal complex having a bifunctional ligand, a carbonyl compound, or the like introduced via a linker in the structure of the derivative may also be used. Here, the bifunctional ligand is preferably polyaminopolycarboxylic acid. The polyaminocarboxylic acid is selected from the group consisting of ethylenediaminetetraacetic acid (EDTA), diethylenetriaminepentaacetic acid (DTPA), diethylenetriamine pentaacetate bismethylamide (DTPA-BMA), 1,4,7,10-tetraazacyclododecane-1,4,7,10-tetraacetic acid (DOTA), and derivatives of these compounds. The bifunctional ligand other than polyaminopolycarboxylic acid is selected from, for example, the group consisting of 6-hydrazinonicotinamide (HYNIC) and derivatives thereof.

A complex useful as a diagnostic agent for nuclear magnetic resonance containing a metal complex of a bifunctional ligand having, as a metal component, a metal ion exhibiting paramagnetic property due to an unpaired electron in the inner shell, for example, a paramagnetic ion of a metal element selected from the group consisting of Co, Mn, Cu, Cr, Ni, V, Au, Fe, Eu, Gd, Dy, Tb, Ho and Er, may also be used. Also, a complex useful as an X-ray diagnostic agent containing a metal complex of a bifunctional ligand having, as a metal component, a metal ion of an X-ray absorbing metal element, i.e., an atom having an atomic number of 20 or more, for example a metal element selected from the group consisting of Re, Sm, Ho, Lu, Pm, Y, Bi, Pb, Os, Pd, Gd, La, Au, Yb, Dy, Cu, Rh, Ag, and Ir, may also be used. Also, a complex useful as a radioactive diagnostic agent containing a metal complex of a bifunctional ligand having, as a metal component, a metal ion of a radionuclide, for example a radionuclide selected from the group consisting of $^{47}Sc$, $^{52}mMn$, $^{55}Co$, $^{62}Cu$, $^{64}Cu$, $^{67}Cu$, $^{67}Ga$, $^{68}Ga$, $^{72}As$, $^{72}Se$, $^{73}Se$, $^{75}Se$, $^{76}As$, $^{95}Tc$, $^{99}mTc$, $^{105}Rh$, $^{109}Pd$, $^{111}In$, $^{153}Sm$, $^{177}Lu$, $^{186}Re$, $^{188}Re$, $^{198}Au$, $^{199}Au$, $^{201}Tl$, $^{211}At$, and $^{212}Bi$ may also be used.

Target diseases of the diagnostic agent of the present invention are diseases involving nitroimidazole susceptible microorganisms or diseases suspicious of infection with these microorganisms. For in vitro diagnosis, blood, urine, sputum, tissues, and the like are used.

Examples thereof include infectious diseases caused by obligate anaerobes or diseases suspicious of infection with obligate anaerobes, particularly, brain abscess, respiratory tract infection, infection in the otorhinolaryngological area, intraperitoneal infection, necrotizing fasciitis, trauma and intraoral infection, or postoperative infections in large intestine, uterus, mouth, etc., and in the periphery of these organs, and fever of unknown origin.

Also, examples thereof include infectious diseases caused by acid-fast bacteria, particularly Mycobacterium tuberculosis infection or diseases suspicious of Mycobacterium tuberculosis infection, particularly diseases caused by infection with Mycobacterium tuberculosis in the resting stage of division, for example latent Mycobacterium tuberculosis infection. Also, the diagnostic agent of the present invention is effective for diagnosis of latent tuberculosis in patients

with HIV and patients with rheumatism being treated with biologics, who are at high risk of developing tuberculosis.

Also, examples thereof include diseases involving microorganisms other than those described above or diseases suspicious of infection with those microorganisms, for example microaerophilic bacterial infection, particularly gastritis, gastric ulcer, duodenal ulcer, gastric cancer, and MALT lymphoma caused by Helicobacter pylori. Examples of the disease thereof also include protozoa infections, particularly genitourinary tract infection with Trichomonas, intestinal amebiasis and extraintestinal amebiasis caused by Entamoeba histolytica, and giardiasis caused by Giardia lamblia.

When the diagnostic agent for infectious diseases of the present invention is used as an in vitro diagnostic agent, the aforementioned compounds or the labeled forms thereof may be detected in blood, urine, sputum, tissues, and the like. Also, when the diagnostic agent for infectious diseases of the present invention is used as an in vivo diagnostic agent, the aforementioned compounds or the labeled forms thereof may be administered to the patients in need thereof, followed by detection of the compounds or the labeled forms.

When the diagnostic agent for infectious diseases of the present invention is used as an in vivo diagnostic agent, the dosage form may be selected in accordance with the intended use and target disease. For example, it can be administered orally, intravenously, intra-arterially, intramuscularly, subcutaneously, intracutaneously, intra-articularly, and intrasynovially and it is most suitably administered by intravenous injection. For example, when the diagnostic agent for infectious diseases of the present invention is a radioactive agent, the dose is appropriately determined based on the weight, age, and sex of the patient and various conditions of an imaging device suitable for detection (such as PET, SPECT, MRI, ESR, NIRS, and X-ray CT). When the aforementioned imidazole derivative is used as a diagnostic imaging agent, for example, it is preferably administered one to 24 hours before imaging.

The diagnostic agent for infectious diseases of the present invention can be prepared by mixing the nitroimidazole derivative with pharmaceutically acceptable additives. For example, the diagnostic agent for infectious diseases of the present invention can be prepared by adding stabilizing agents such as propylene glycol; pH adjusters such as acids and bases; buffers such as phosphate buffer; isotonic agents such as physiological saline, emulsifiers/dispersants, excipients, binders, coating agents, stabilizers, sugars such as mannitol, and lyophilization aids such as amino acids. Also, in the case of a nitroimidazole derivative composed of a metal complex, reducing agents can be added for production of a metal complex. Examples of the reducing agent include tin(II) chloride, tin(II) tartrate, other tin(II) compounds, metallic tin, a mixture of ascorbic acid and ferric chloride, sodium dithionite, and ferrous sulfate. Among these reducing agents, tin reducing agents, particularly tin(II) chloride is preferred.

In order to screen for a therapeutic agent for infectious diseases caused by microorganisms by the screening method of the present invention, binding of a control nitroimidazole derivative to the microorganisms may be detected in the presence of a test substance either in vivo or in vitro.

For example, for in vitro screening, a method of detecting binding of a control nitroimidazole derivative to the microorganisms in the presence of a test substance and a method of detecting the microorganisms in the presence of a test substance and a control nitroimidazole derivative are performed.

For example, for in vivo screening, a control nitroimidazole derivative and a test substance may be administered to an animal infected with microorganisms and the presence or absence of surviving microorganisms may be detected in the animals. If the test substance inhibits the binding of a control nitroimidazole derivative to the microorganisms, then the test substance is found to be useful as a therapeutic agent for infectious diseases caused by the target microorganisms.

[Examples]

Hereinbelow, the present invention will be described in detail with reference to Examples; however, the present invention is not limited in any way by these Examples.

Example 1

Microorganism susceptible to and microorganism non-susceptible to metronidazole, which is an antimicrobial drug having a nitroimidazole structure, were compared. As the microorganisms, Bacteroides fragilis (ATCC25285) and E.coli (ATCC25922) were used. Bacteroides fragilis is an obligate anaerobe, which is a representative metronidazole susceptible microorganism. E.coli is a facultative anaerobe, which is a non-metronidazole susceptible microorganism. As to the culture method of each microorganism, Bacteroides fragilis and E.coli were cultured in a modified GAM medium and a Mueller-Hinton medium, respectively, at 37°C for one to two days before use in the experiments. Anaerobic culture was performed in the Anaero Pack (MITSUBISHI GAS CHEMICAL COMPANY, INC.). Phosphate buffered saline subjected to high pressure steam sterilization treatment, followed by rapid cooling for deaeration was used. Also, the microorganisms were handled in a nitrogen gas-filled environment. When operation was carried out in air, the container containing the microorganisms was hermetically closed to avoid dissolution of oxygen in air.

Experiments were performed using the following nitroimidazole derivatives:

Metronidazole

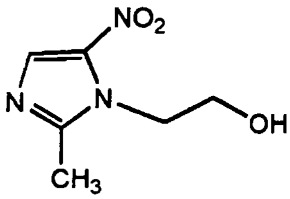

Fluoromisonidazole (FMISO)

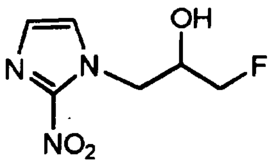

(wherein, F represents $^{18}F$ or $^{19}F$.)

Compound 1 : [$^{125}I$]N1-[2-(2-methyl-5-nitro-1H-1-imidazolyl)ethyl]-3-iodobenzamide

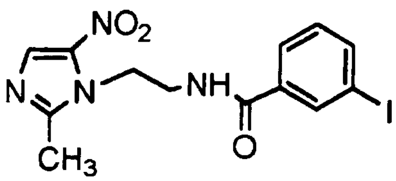

(wherein, I represents $^{125}I$.)

Compound 2: N1-[2-(2-nitro-1H-1-imidazolyl)ethyl]-3-iodobenzamide

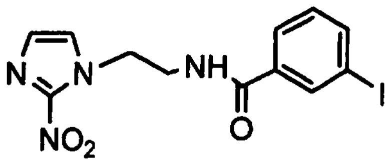

(wherein, I represents $^{125}I$ or $^{127}I$.)

Compound 3: [$^{67}Ga$]Ga-DOTA-(metronidazole)$_2$

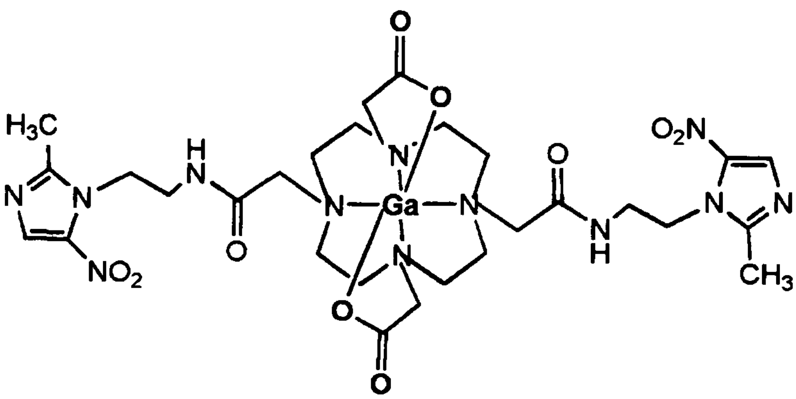

(wherein, Ga represents $^{67}$Ga.)

An in vitro assay was performed using metronidazole, which is frequently used as a therapeutic agent, and FMISO, which is often used in research for diagnostic imaging agents, both of which are representative examples of nitroimidazole derivatives. Metronidazole and [$^{19}$F]FMISO were obtained from Sigma-Aldrich Co. LLC. and ABX advanced biochemical compounds, respectively.

Into test tubes containing phosphate buffered saline, 100 μM metronidazole, 70 μM [$^{19}$F]FMISO and each bacterium were added while ice-cooling so as to achieve a McFarland standard of 6.0. The test tubes were stirred and then incubated for each predetermined time period in a water bath of 37°C. After incubation, the test tubes were immediately cooled on ice and then centrifuged (3000 rpm, 20 minutes, 4°C) to separate bacteria to obtain supernatants. The supernatants thus obtained were filtered through a filtration sterilization filter (pore size of 200 nm), and the absorbance was measured at 318 nm for metronidazole and at 322 nm for [$^{19}$F]FMISO (absorbance of supernatant). Also, the absorbance of supernatants obtained by incubating solutions without added nitroimidazole derivatives, i.e., solutions containing only bacteria, for each predetermined time period was used as the background absorbance.

The rate of uptake (%) was obtained by the following formula.

$$\text{The rate of uptake (\%)} = 100 - (B / A \times 100)$$

A: Absorbance at the final concentration of nitroimidazole derivative
B: (Absorbance of supernatant) - (background absorbance)

The amount of viable bacteria at each incubation time was obtained by the quantitative culture method, and the rate of uptake per $10^8$-colony forming unit (CFU) was calculated. The results of metronidazole and [$^{19}$F]FMISO are shown in Figures 1 and 2, respectively.

Metronidazole, which is a representative nitroimidazole derivative, was taken up in a large amount by the obligate anaerobe Bacteroides fragilis (B.fragilis), whereas it was taken up in little amount by the facultative anaerobe E.coli. Based on the above results, the present inventors found that metronidazole was taken up in a large amount by metronidazole susceptible microorganisms. Utilization of this new finding enables application of metronidazole to an in vitro diagnostic agent and the like. For example, when a specimen is anaerobically cultured, both facultative anaerobes and obligate anaerobes will grow, and for confirmation of obligate anaerobes from these bacteria, complicated operations will be necessary. In this respect, the presence of obligate anaerobes can be easily confirmed and diagnosis can be rapidly made by utilizing these accumulation characteristics of the nitroimidazole derivative.

The present inventors found that, similarly to metronidazole, [$^{19}$F]FMISO was taken up in a greater amount by obligate anaerobes than by facultative anaerobes. Because [$^{19}$F]FMISO contains $^{19}$F having nuclear spin, it can be detected by a nuclear magnetic resonance imaging device or similar means. That is, nitroimidazole derivatives having nuclear spin can be utilized as a diagnostic agent for a non-invasive diagnostic method for infectious diseases caused by microorganisms susceptible to antimicrobial drugs having a nitroimidazole structure by a nuclear magnetic resonance imaging device or similar means.

Example 2

Compound 1: Synthesis of [$^{125}$I]N1-[2-(2-methyl-5-nitro-1H-1-imidazolyl)ethyl]-3-iodobenzamide

Synthesis of 2-(2-methyl-5-nitro-1H-1-imidazolyl)-1-ethylamine hydrochloride

Into a solution of 2-methyl-4-nitroimidazole (1.136 g) in dimethylformamide (25 mL), N-(2-bromoethyl)phthalimide (2.367 g) and potassium carbonate (1.284 g) were added, followed by stirring at 110°C for three hours and filtration. After distilling off the solvent in the filtrate, water was added to the residue and the resulting solution was stored in a refrigerator. The precipitate thus formed was collected by filtration, which was washed and then dried under reduced pressure. Ethanol was added to the compound thus obtained, to which hydrazine hydrate (0.38 mL) was added, followed by reflux for three hours. Ethanol was added and the resulting product was stored in a refrigerator. The precipitate thus formed was collected by filtration and the solvent was distilled off. To the residue thus obtained, an aqueous solution of hydrochloric acid was added, and the precipitate thus formed was filtered. After distilling off the solvent, the resulting residue was dissolved in methanol, to which ethyl acetate was added. The precipitate thus formed was collected by filtration to give the title compound (0.581 g).
$^1$H-NMR (400 MHz, $D_2O$) δ: 2.47 (3H, s), 3.47-3.51 (2H, t, J=6.1 Hz), 4.40-4.43 (2H, t, J=6.3 Hz), 8.19 (1H, s).

Synthesis of N1-[2-(2-methyl-5-nitro-1H-1-imidazolyl)ethyl]-3-(1,1,1-tributylstannyl)benzamide

Into a solution of 2-(2-methyl-5-nitro-1H-1-imidazolyl)-1-ethylamine hydrochloride (17 mg) in dimethylformamide (3 mL), triethylamine (34 μL) and a solution of 2,5-dioxotetrahydro-1H-1-pyrrolyl-3-(1,1,1-tributylstannyl)benzoate (50 mg) in dimethylformamide (2 mL) were added, followed by stirring at room temperature overnight. After distilling off the solvent, dichloromethane was added to the residue, followed by washing with water. The resulting organic layer was dried over anhydrous sodium sulfate and after that the solvent was distilled off. The resulting residue was purified by silica gel column chromatography to give the title compound (38.4 mg).
$^1$H-NMR (400 MHz, $CDCl_3$) δ: 0.83-1.49 (27H, m), 1.84 (3H, s), 3.81-3.85 (2H, m), 4.16-4.19 (2H, t, J=5.1), 7.30-7.33 (1H, t, J=7.6 Hz), 7.57 (1H, d), 7.69 (1H, d), 7.83 (1H, d), 7.89 (1H, s).
ESI-MS (m/z): 565 ($M-H^+$)

Synthesis of N1-[2-(2-methyl-5-nitro-1H-1-imidazolyl)ethyl]-3-iodobenzamide

Into a solution of 2-(2-methyl-5-nitro-1H-1-imidazolyl)-1-ethylamine hydrochloride (50 mg) in dimethylformamide (5 mL), triethylamine (100 μL) and a solution of 2,5-dioxotetrahydro-1H-1-pyrrolyl-3-iodobenzoate (100 mg) in dimethylformamide (3 mL) were added, followed by stirring at room temperature overnight. After distilling off the solvent, dichloromethane was added to the residue, followed by washing with water. The resulting organic layer was dried over anhydrous sodium sulfate and after that the solvent was distilled off. The resulting residue was then dissolved in dichloromethane and impurities were filtered off to give the title compound (63.4 mg).
$^1$H-NMR (400 MHz, DMSO-$d_6$) δ: 2.30 (3H, s), 3.58-3.63 (2H, m), 4.15-4.18 (2H, t, J=5.3), 7.25-7.29 (1H, t, J=7.8 Hz), 7.75-7.78 (1H, m), 7.79-7.90 (1H, m) 8.09 (1H, d).8.29 (1H, s).
ESI-MS (m/z): 400 ($M^+$)

Synthesis of [$^{125}$I] N1-[2-(2-methyl-5-nitro-1H-1-imidazolyl)ethyl]-3-iodobenzamide

Into a mixed solution of a solution of N1-[2-(2-methyl-5-nitro-1H-1-imidazolyl)ethyl]-3-(1,1,1-tributylstannyl) benzamide (0.5 mg/mL) in methanol (20 μL), 70 μL of a sodium phosphate buffered solution pH5.5), and 10 μl of a sodium iodide[$^{125}$I] solution (5 mCi), 20 μl of an aqueous solution of sodium p-toluenesulfonchloramide was added. After leaving the resulting mixture for two minutes, 100 μl of an aqueous solution of sodium disulfite was added to terminate the reaction. The resulting reaction mixture was separated and purified by reverse phase HPLC (Mightsil RP-18, 6.0 x 150 mm). At this time, benzyl alcohol was added as a stabilizing agent. Fractionated solutions were then distilled off under reduced pressure and an adequate amount of water was added, and after that the resulting solution was filtered to prepare a solution of target substance. TLC analysis showed a radiochemical purity of 95% or higher and a specific radioactivity of approximately 2200 Ci/mmol.

Compound 2: Synthesis of [$^{125}$I]N1-[2-(2-nitro-1H-1-imidazolyl)ethyl]-3-iodobenzamide

Synthesis of 2-(2-1H-1-imidazolyl)-1-ethylamine hydrochloride

Into a solution of 2-nitroimidazole (0.255 g) in dimethylformamide (20 mL), N-(2-bromoethyl)phthalimide (0.607 g) and potassium carbonate (0.330 g) were added, followed by stirring at 110°C for three hours and subsequently filtration. After distilling off the solvent in the filtrate, water was added to the residue and the resulting solution was stored in a refrigerator. The precipitate thus formed was collected by filtration, which was washed and then dried under reduced pressure. Ethanol was added to the compound thus obtained, to which hydrazine hydrate (0.22 mL) was added, followed by reflux for three hours. Ethanol was added, the resulting product was stored in a refrigerator, then the precipitate thus formed was filtered and the solvent was distilled off. To the residue thus obtained, an aqueous solution of hydrochloric acid was added, and the precipitate thus formed was filtered. After distilling off the solvent, the resulting residue was dissolved in methanol, to which ethyl acetate was added. The precipitate thus formed was collected by filtration to give the title compound (0.284 g).
$^1$H-NMR (400 MHz, $D_2O$)
δ: 3.57-3.60 (2H, t, J=6.3 Hz), 4.81-4.84 (2H, t, J=6.3 Hz), 7.26 (1H, d), 7.55 (1H, d).

Synthesis of N1-[2-(2-nitro-1H-1-imidazolyl)ethyl]-3-(1,1,1-tributylstannyl)benzamide

Into a solution of 2-(2-nitro-1H-1-imidazolyl)-1-ethylamine hydrochloride (16.3 mg) in dimethylformamide (3 mL), triethylamine (34 μL) and a solution of 2,5-dioxotetrahydro-1H-1-pyrrolyl-3-(1,1,1-tributylstannyl)benzoate (50 mg) in dimethylformamide (1 mL) were added, followed by stirring at room temperature overnight. After distilling off the

solvent, dichloromethane was added to the residue, followed by washing with water. The resulting organic layer was dried over anhydrous sodium sulfate and after that the solvent was distilled off. The resulting residue was purified by silica gel column chromatography to give the title compound (26.8 mg).
$^1$H-NMR (400 MHz, $CDCl_3$)
δ: 0.85-1.54 (27H, m), 3.88-3.93 (2H, m), 4.71-4.74 (2H, t, J=5.8), 6.84 (1H, s), 7.02 (1H, d), 7.32-7.36 (1H, m) 7.59 (1H, d), 7.64 (1H, d), 7.86 (1H, d). ESI-MS (m/z): 551 ($M-H^+$)

Synthesis of N1-[2-(2-nitro-1H-1-imidazolyl)ethyl]-3-iodobenzamide

Into a solution of 2-(2-nitro-1H-1-imidazolyl)-1-ethylamine hydrochloride (23.3 mg) in dimethylformamide (5 mL), triethylamine (50.3 μL) and a solution of 2,5-dioxotetrahydro-1H-1-pyrrolyl-3-iodobenzoate (50 mg) in dimethylformamide (1 mL) were added, followed by stirring at room temperature overnight. After distilling off the solvent, dichloromethane was added to the residue, followed by washing with water. The resulting organic layer was dried over anhydrous sodium sulfate and after that the solvent was distilled off. The resulting residue was then dissolved in dichloromethane and impurities were filtered off to give the title compound (21.8 mg).
$^1$H-NMR (400 MHz, DMSO-$d_6$)
δ: 3.66-3.70 (2H, m), 4.54-4.57 (2H, t, J=6.3), 7.12 (1H, t, J=0.98 Hz), 7.24-7.28 (1H, t, J=7.8 Hz), 7.52 (1H, s), 7.71-7.73 (1H, m), 7.86-7.89 (1H, m) 8.04 (1H, d).
ESI-MS (m/z): 387 ($M-H^+$)

Synthesis of [$^{125}$I]N1-[2-(2-nitro-1H-1-imidazolyl)ethyl]-3-iodobenzamide

Into a mixed solution of a solution of N1-[2-(2-nitro-1H-1-imidazolyl)ethyl]-3-(1,1,1-tributylstannyl)benzamide (0.5 mg/mL) in methanol (20 μl), 70 μl of a sodium phosphate buffered solution pH 5.5), and 10 μl of a sodium iodide[$^{125}$I] solution (5 mCi), 20 μl of an aqueous solution of sodium p-toluenesulfonchloramide was added. After leaving the resulting mixture for two minutes, 100 μl of an aqueous solution of sodium disulfite was added to terminate the reaction. The resulting reaction mixture was separated and purified by reverse phase HPLC (Mightsil RP-18, 6.0 x 150 mm). At this time, benzyl alcohol was added as a stabilizing agent. Fractionated solutions were then distilled off under reduced pressure and an adequate amount of water was added, and after that the resulting solution was filtered to prepare a solution of target substance. TLC analysis showed a radiochemical purity of 95% or higher and a specific radioactivity of approximately 2200 Ci/mmol.

Synthesis of [$^{18}$F]FMISO

The $^{18}$F ions produced by the $^{18}$O(p, n)$^{18}$F reaction using $H_2{}^{18}O$ as a target were allowed to pass through and adsorb to QMA cartridge, which is a strongly basic anion exchange resin, and then eluted with 0.6 mL of an aqueous solution of potassium carbonate (4.56 mg/mL) into a reaction container. To this, 0.6 mL of a solution of Kryptofix 222 in anhydrous acetonitrile (20 mg/mL) was added, followed by heating under a nitrogen stream to distill off the solvent. Further, 1 mL of anhydrous acetonitrile was added, followed by heating under a nitrogen stream and by azeotropic drying. Subsequently, 0.5 mL of a solution of 1-(2'-nitro-1'-imidazolyl)-2-O-tetrahydropyranyl-3-O-toluenesulfonylpropanediol in anhydrous acetonitrile (10 mg/mL) was added, followed by heating at 100°C for 10 minutes to carry out fluorination reaction. The resulting reaction solution was then concentrated to dryness, to which hydrochloric acid was added and hydrolysis was performed. Subsequently, sodium acetate was added for neutralization, and then unreacted $^{18}$F ions were removed with a $C_{18}$ cartridge. Thereafter, the resulting product was separated and purified by reverse phase HPLC (YMC-Pack ODSA A-323, 10 mm i.d. x 250 mm). Fractionated solutions were passed through the QMA cartridge and the resulting eluate was collected in a flask of a rotary evaporator (containing an ascorbic acid injection solution). After distilling off the solvent, the resulting product was dissolved in physiological saline and then filtered, whereby a solution of a target substance was prepared.

Into test tubes containing phosphate buffered saline, 26 to 37 KBq of radionuclide-labeled nitroimidazole derivatives and each bacterium were added while ice-cooling so as to achieve a McFarland standard of 1.0 to 2.0. The test tubes were stirred and then incubated for each predetermined time period in a water bath of 37°C. After incubation, ice-cooled phosphate buffered saline was immediately added and bacteria were separated by centrifugation (3000 rpm, 20 minutes, 4°C). After discarding the supernatant, ice-cooled phosphate buffered saline was added to the separated bacteria, followed by centrifugation. This operation was repeated twice, after which the radioactivity (cpm) of the separated bacteria was measured with a gamma counter. The amount of viable bacteria at each incubation time was obtained by the quantitative culture method, and the rate of uptake per $10^8$-colony forming unit (CFU) was calculated. The results of Compound 1, Compound 2, and [$^{18}$F]FMISO are shown in Figures 3, 4, and 5, respectively.

The present inventors found that Compound 1, Compound 2, and [$^{18}$F]FMISO were taken up in a greater

amount by obligate anaerobes than by facultative anaerobes. Based on these results, for example, by labeling a nitroimidazole derivative with a radionuclide according to the intended use, it can be utilized as an in vitro or in vivo radioactive diagnostic agent for infectious diseases caused by microorganisms susceptible to antimicrobial drugs having a nitroimidazole structure.

Example 3

In order to confirm that the nitroimidazole derivative of the present invention is taken up by a similar mechanism to that for metronidazole, effects provided by metronidazole were studied. When the experimental method of Example 2 was carried out by adding metronidazole to achieve 1.5 mM before incubation at 37°C, the rates of uptake of Compounds 1 and 2 were reduced by 71% and 42%, respectively. That is, it was confirmed that the nitroimidazole derivative of the present invention was taken up by a similar mechanism to that for metronidazole.

Example 4

Compound 3: Synthesis of $[^{67}Ga]$Ga-DOTA-(metronidazole)$_2$

Synthesis of 1,7-bis(tert-butoxycarbonyl)-1,4,7,10-tetraazacyclododecane

Cyclen (1.05 g) was dissolved in chloroform (50 mL), to which N-(tert-butoxycarbonyloxy)succinimide (2.62 g) was added, followed by stirring at room temperature. After 48 hours of stirring, the solvent was distilled off under reduced pressure. The resulting residue was dissolved in chloroform (50 mL), washed with an aqueous solution of sodium hydroxide, and dried over sodium sulfate. The solvent was distilled off under reduced pressure and the resulting product was dried in a vacuum to give the title compound (2.27 g).

Synthesis of 1,7-bis(tert-butoxycarbonyl)-4,10-bis(benzyloxycarbonylmethyl)-1,4,7,10-tetraazacyclododecane

Into anhydrous acetonitrile (55 mL), 1,7-bis(tert-butoxycarbonyl)-1,4,7,10-tetraazacyclododecane (2.40 g) was dissolved, to which benzylbromoacetate (2.4 mL) and potassium carbonate (2.13 g) were added, followed by stirring at room temperature. After 24 hours of stirring, potassium carbonate was removed and the solvent was distilled off from the filtrate under reduced pressure. The resulting residue was separated and purified by silica gel column to give the title compound (3.92 g).

Synthesis of 4,10-(benzyloxycarbonylmethyl)-1,4,7,10-tetraazacyclododecane

Into dichloromethane (1.4 mL), 1,7-bis(tert-butoxycarbonyl)-4,10-bis(benzyloxycarbonylmethyl)-1,4,7,10-tetraazacyclododecane (49.2 mg) was dissolved, to which trifluoroacetic acid (1.4 mL) was added. After 90 minutes of stirring, the solvent was distilled off under reduced pressure. The resulting product was dissolved in an aqueous solution of sodium hydroxide (15 mL), extracted with diethyl ether, and dried over sodium sulfate. The solvent was distilled off under reduced pressure and the resulting product was dried in a vacuum to give the title compound (33.4 mg).

Synthesis of 1,7-bis(benzyloxycarbonylmethyl)-4,10-bis(tert-butoxycarbonylmethyl)-1,4,7,10-tetraazacyclododecane

Into anhydrous acetonitrile (40 mL), 4,10-(benzyloxycarbonylmethyl)-1,4,7,10-tetraazacyclododecane (2.32 g) was dissolved, to which tert-butyl bromoacetate (1.78 mL) and potassium carbonate (1.64 g) were added, followed by stirring at room temperature. After 24 hours, potassium carbonate was removed and the solvent was distilled off from the filtrate under reduced pressure. The resulting residue was separated and purified by silica gel column to give the title compound (3.20 g).

Synthesis of 4,10-bis(tert-butoxycarbonylmethyl)-1,4,7,10-tetraazacyclododecane-1,7-diacetic acid

Into anhydrous ethanol (20 mL), 1,7-bis(benzyloxycarbonylmethyl)-4,10-bis(tert-butoxycarbonylmethyl)-1,4,7,10-tetraazacyclododecane (900 mg) was dissolved, to which 10% palladium on carbon (100 mg) was added, followed by stirring under a hydrogen stream at 0.2 MPa at room temperature. After 60 hours of stirring, palladium on carbon was removed. The solvent was then distilled off from the filtrate under reduced pressure and the resulting product was dried in a vacuum to give the title compound (798 mg).

Synthesis of 2-(2-methyl-5-nitroimidazol-1-yl)ethyl methanesulfonate

Into anhydrous dichloromethane (40 mL), metronidazole (2.0 g) was dissolved, to which triethylamine (2.4 mL) and methanesulfonyl chloride (1.1 mL) were added, followed by stirring at room temperature under an argon stream. After four hours, the solid thus precipitated was collected by filtration and dried in a vacuum to give the title compound (2.64 g).

Synthesis of 1-(2-azide-ethyl)-2-methyl-5-nitro-1H-imidazole

Into dimethylformaldehyde (30 mL), 2-(2-methyl-5-nitroimidazol-1-yl)ethyl methanesulfonate (2.36 g) was dissolved, to which sodium azide (0.73 g) was added, followed by stirring at 100°C. After two hours, the resulting solution was returned to room temperature, followed by further stirring for two hours. Saturated saline was then added, and the resulting solution was extracted with ethyl acetate, and then dried over sodium sulfate. The solvent was distilled off under reduced pressure and the resulting product was dried in a vacuum to give the title compound (1.80 g).

Synthesis of 2-(2-methyl-5-nitroimidazol-1-yl)ethylamine dihydrochloride

Into anhydrous tetrahydrofuran (40 mL), 1-(2-azide-ethyl)-2-methyl-5-nitro-1H-imidazole (2.93 g) was dissolved, to which triphenylphosphine (4.87 g) was added, followed by stirring at room temperature. After 20 hours, concentrated hydrochloric acid was added, followed by heating to reflux. After five hours, the solvent was distilled off under reduced pressure, and the resulting residue was dissolved in purified water and washed with ethyl acetate. The solvent was distilled off under reduced pressure and the resulting product was dried in a vacuum. The solid thus obtained was recrystallized using methanol to give the title compound (2.69 g).

Synthesis of 4,10-bis{[2-(2-methyl-5-nitroimidazol-1-yl)ethylcarbamoyl]methyl}-1,7-bis(tert-butoxycarbonylmethyl)-1,4,7,10-tetraazacyclododecane

Into anhydrous dichloromethane (5.0 mL) and anhydrous dimethylformaldehyde (1.0 mL), 4,10-bis(tert-butoxycarbonylmethyl)-1,4,7,10-tetraazacyclododecane-1,7-diacetic acid (60.0 mg) was dissolved, to which anhydrous triethylamine (132 μL), ethyl-(dimethyl)-carbodiimide (89.4 mg), hydroxybenzotriazole (71.4 mg), 2-(2-methyl-5-nitroimidazol-1-yl)ethylamine dihydrochloride (112.8 mg) were added, followed by stirring under an argon stream. After 48 hours, chloroform was added, and the resulting mixture was vigorously washed with purified water. The solvent was then distilled off under reduced pressure and the residue thus obtained was recrystallized using methanol to give the title compound (32.3 mg).

Synthesis of 4,10-bis{[2-(2-methyl-5-nitroimidazol-1-yl)ethylcarbamoyl]methyl}-1,4,7,10-tetraazacyclododecane-1,7-diacetic acid

Into concentrated hydrochloric acid, 4,10-bis{[2-(2-methyl-5-nitroimidazol-1-yl)ethylcarbamoyl]methyl}-1,7-bis(tert-butoxycarbonylmethyl)-1,4,7,10-tetraazacyclododecane (17.0 mg) was dissolved, followed by stirring at room temperature. After two hours of stirring, the solvent was distilled off under reduced pressure. The resulting residue was dissolved in a small amount of methanol, solidified with diethyl ether, and then dried in a vacuum to give the title compound (14.0 mg).

Synthesis of [$^{67}$Ga]Ga-DOTA-(metronidazole)$_2$

Into a 0.2 M ammonium acetate buffer (pH 5.8) (198 μL), 4,10-bis{[2-(2-methyl-5-nitroimidazol-1-yl)ethylcarbamoyl]methyl}-1,4,7,10-tetraazacyclododecane-1,7-diacetic acid (7 μg) was dissolved, to which [$^{67}$Ga]gallium chloride (FUJIFILM RI Pharma Co., Ltd.) (2 μL) was added, followed by heating at 95°C for 60 minutes. The resulting reaction mixture was separated and purified by reverse phase column (COSMOCIL 5C18-PAQ 4.6 x 250 mm). After separation and purification, the solvent of fractionated solution was distilled off under reduced pressure to dryness.

An in vitro assay was performed in a similar manner to the assay of Example 2 by using Compound 3, a nitroimidazole derivative composed of a metal complex having therein a bifunctional ligand, a carbonyl compound, or the like introduced as a linker. Also, the rate of uptake of Compound 3 by each predetermined amount of viable Bacteroides fragilis while heating at 37°C for 120 minutes was obtained. The predetermined amount of viable Bacteroides fragilis ranged from 0.43 x $10^8$ CFU/mL to 6.75 x $10^8$ CFU/mL.

The present inventors found that Compound 3 was taken up in a greater amount by obligate anaerobes than by facultative anaerobes (Figure 6). Also, the rate of uptake of Compound 3 increased in a manner correlated with the

number of viable obligate anaerobes (Figure 7).

It was confirmed that the nitroimidazole derivative composed of a metal complex having therein a bifunctional ligand, a carbonyl compound, or the like introduced as a linker could be used as an in vitro or in vivo radioactive diagnostic agent capable of measuring the number of viable microorganisms susceptible to antimicrobial drugs having a nitroimidazole structure. That is, such a nitroimidazole derivative can be utilized as a radioactive diagnostic agent, an imaging agent for nuclear magnetic resonance, an X-ray imaging agent, and the like by changing the metal complex according to the intended use.

Example 5

An in vivo assay was performed using a rat subcutaneous infection model. As the nitroimidazole derivative, Compound 1 or Compound 2 was used. As the microorganism, two strains of Bacteroides fragilis (ATCC25285, NCTC10581), which is susceptible to metronidazole, or E.coli (ATCC25922), which is not susceptible to metronidazole, was used. A 6-week-old male SD rat was administered with 5 x $10^9$ cfu/0.1 ml bacteria suspended in physiological saline subcutaneously into the left thigh, and then served for evaluation four days later. Approximately 0.74 MBq of Compound 1 or Compound 2 was administered via the vena jugularis externa, and three hours later, blood was collected and tissues were excised. Also, for evaluation of inflammation in the infection model, approximately 0.15 MBq of [$^{67}$Ga]gallium citrate (FUJIFILM RI Pharma Co., Ltd.), which is a radioactive diagnostic agent for inflammation, was administered via the vena jugularis externa 6.5 hours before blood collection. The viable bacteria in the infected tissue was confirmed by smearing a part of the infected skin tissue on a medium and culturing it. Radioactivity (cpm) of each of $^{67}$Ga and $^{125}$I was measured in blood, in the infected skin site, and in the non-infection site on the skin. Radioactivity of $^{125}$I was measured after attenuating the radioactivity of $^{67}$Ga. The amount accumulated in each tissue and %ID/g (the ratio of radioactivity per tissue weight relative to the radioactivity administered) were calculated.

The accumulation of [$^{67}$Ga]gallium citrate in the rat subcutaneous infection model was found as follows; Bacteroides fragilis (ATCC25285): 1.75 $\pm$ 0.2, Bacteroides fragilis (NCTC10581): 1.89 $\pm$ 0.4, and E.coli: 1.73 $\pm$ 0.7 in terms of the value of the amount accumulated in the infected skin site/blood, and the amount accumulated in the non-infected skin site/blood was calculated as 0.41 $\pm$ 0.06, showing that inflammation was caused by bacteria. As a result of the inflammation evaluation by [$^{67}$Ga]gallium citrate, there was no difference in the inflammation caused by each bacterium.

The results of the rat subcutaneous infection model were shown in Figure 8. Compared to the site of infection with metronidazole non-susceptible E.coli, selective accumulation in the site of infection with metronidazole susceptible Bacteroides fragilis was observed. This in vivo assay also provided the results consistent with the in vitro assay. Based on this Example, the nitroimidazole derivative can be utilized as, for example, a diagnostic agent for diagnostic imaging of the infectious disease caused by microorganisms susceptible to antimicrobial drugs having a nitroimidazole structure.

Example 6

Accumulation of Compound 1 or 2 in an inflammation model not involving viable microorganisms was evaluated. Approximately half of the content of the cecum is composed of bacteria or dead bodies thereof, and most of the constituent bacteria are obligate anaerobes. The content of the rat cecum was diluted 8-fold with a GAM medium and filtered through gauze, and the resulting cecal content was sterilized by autoclaving (121°C, two hours). The resulting cecal content was confirmed to have been sterilized by culture method. The inflammation model was administered with 0.1 mL of the sterilized cecal content subcutaneously into the thigh, and served for the study four days later. Approximately 0.74 MBq of Compound 1 or Compound 2 was administered via the vena jugularis externa, and three hours later, blood was collected and tissues were excised. Also, for confirmation of inflammation, approximately 0.15 MBq of [$^{67}$Ga]gallium citrate (FUJIFILM RI Pharma Co., Ltd.), which is a radioactive diagnostic agent for inflammation, was administered via the vena jugularis externa 6.5 hours before blood collection. Radioactivity (cpm) of each of $^{67}$Ga and $^{125}$I was measured in blood and in the skin on the inflammation side. Radioactivity of $^{125}$I was measured after attenuating the radioactivity of $^{67}$Ga. The amount accumulated in each tissue and %ID/g (the ratio of radioactivity per tissue weight relative to the radioactivity administered) were calculated.

Accumulation of [$^{67}$Ga]gallium citrate in the sterilized cecal content-subcutaneous inflammation model was found to be 1.61 $\pm$ 0.16 in terms of the value of the amount accumulated in the inflammatory skin site/blood, exhibiting greater accumulation of [$^{67}$Ga]gallium citrate than that in normal skin. From this result, inflammation was confirmed.

Accumulation of Compound 1 was found to be 0.85 $\pm$ 0.25 and that of Compound 2 was found to be 0.73 $\pm$ 0.11 in terms of the value of the amount accumulated in the inflammatory skin site/blood, exhibiting a lower value than that in the Bacteroides fragilis-infection site. This result confirmed that the nitroimidazole derivative accumulated in viable microorganisms susceptible to antimicrobial drugs having a nitroimidazole structure. The nitroimidazole derivative can be utilized as a novel diagnostic agent, for example, it is useful for short-term use of antimicrobial drugs by non-invasively measuring viable microorganisms in a short period of time.

Example 7

The nitroimidazole derivative has characteristics such that it exerts an antimicrobial activity also on acid-fast bacteria in the resting stage of division (under an anaerobic environment). Uptake of Compound 2, which is a nitroimidazole derivative, by acid-fast bacteria was evaluated. As the nitroimidazole susceptible acid-fast bacteria, attenuated Mycobacterium bovis (BCG, ATCC35734) was used. As the nitroimidazole non-susceptible bacteria, Escherichia coli (E.coli, ATCC25922), which is a facultative anaerobe, was used. Uptake of Compound 2 under an anaerobic environment was compared between BCG and E.coli. BCG was cultured in a Middlebrook 7H9 Broth at 37°C and then filtered through a 50 μm membrane filter to remove a clump of bacteria before use. E.coli (ATCC25922) was prepared by the method of Example 1. This experiment was performed in a phosphate buffered saline (containing 2% DMSO and 0.05% polysorbate 80) deaerated by high pressure steam sterilization treatment (121°C, 15 minutes). Also, bacteria were handled in a nitrogen gas-filled environment. When the operation was carried out in air, the container containing the microorganisms was hermetically closed to avoid dissolution of oxygen in air.

Into test tubes containing phosphate buffered saline, Compound 2 was added at a final concentration of 0.1 mM, and each bacterium was added while ice-cooling so as to achieve a McFarland standard of 3 to 4. The test tubes were stirred and then incubated for each predetermined time period in a water bath of 37°C. After incubation, the test tubes were immediately cooled on ice and bacteria were separated by centrifugation (3000 rpm, 20 minutes, 4°C) to obtain supernatants. The supernatants thus obtained were filtered through a filtration sterilization filter (pore size of 200 nm), and Compound 2 was measured at an absorbance of 325 nm (absorbance of supernatant). Also, the absorbance of supernatants obtained by incubating solutions containing only bacteria without added Compound 2 for each predetermined time period was used as the background absorbance.

The rate of uptake (%) was obtained by the following formula.

$$\text{The rate of uptake (\%)} = 100 - (B / A \times 100)$$

A: Absorbance at the final concentration of nitroimidazole derivative
B: (Absorbance of supernatant) - (background absorbance)

The amount of viable bacteria (colony forming unit) at each incubation time was obtained by the quantitative culture method using a Middlebrook 7H11 agar medium for BCG and a Mueller-Hinton agar medium for E.coli, and the rate of uptake per $10^7$-colony forming unit (CFU) was calculated. The results thus obtained are shown in Figure 9.

The present inventors found that Compound 2, which is a nitroimidazole derivative, was clearly taken up in a greater amount by nitroimidazole derivative susceptible BCG than by non-nitroimidazole derivative susceptible E.coli under an anaerobic environment. Utilizing this new finding, for example, by labeling Compound 2 with a radionuclide according to the intended use, it can be used as an in vivo or in vitro radioactive diagnostic agent for tuberculosis infection. In particular, because Compound 2 can detect Mycobacterium tuberculosis in the resting stage of division, it is effective as a diagnostic imaging agent for determination of the therapeutic efficacy for Mycobacterium tuberculosis in the resting stage of division, a diagnostic imaging agent for latent tuberculosis, and the like.

Example 8

The uptake of FMISO, which is frequently used in research for diagnostic imaging agents, by nitroimidazole susceptible acid-fast bacteria was evaluated. As the nitroimidazole susceptible acid-fast bacteria, BCG (ATCC35734) was used. BCG was cultured in a Middlebrook 7H9 Broth at 37°C and then filtered through a 50 μm membrane filter to remove a clump of bacteria before use. This experiment was performed in a phosphate buffered saline (containing 0.05% polysorbate 80) deaerated by high pressure steam sterilization treatment (121°C, 15 minutes). Also, bacteria were handled in a nitrogen gas-filled environment. When the operation was carried out in air, the container containing the microorganisms was hermetically closed to avoid dissolution of oxygen in air.

Into test tubes containing phosphate buffered saline, [$^{19}$F] FMISO was added at a final concentration of 0.09 mM, and BCG was added so as to achieve a McFarland standard of 3 to 4 while ice-cooling. The test tubes were stirred and then incubated for each predetermined time period in a water bath of 37°C. After incubation, the test tubes were immediately cooled on ice and bacteria were separated by centrifugation (3000 rpm, 20 minutes, 4°C) to obtain supernatants. The supernatants thus obtained were filtered through a filtration sterilization filter (pore size of 200 nm), and [$^{19}$F] FMISO was measured at an absorbance of 322 nm (absorbance of supernatant). Also, the absorbance of supernatants obtained by incubating solutions containing only bacteria without added [$^{19}$F] FMISO for each predetermined time period was used as the background absorbance.

The rate of uptake (%) was obtained by the same method as in Example 7.
The results thus obtained are shown in Figure 10. Also, the results of the uptake of [$^{19}F$] FMISO by E.coli in Example 1 were superimposed in Figure 10 for comparison.

The present inventors found that [$^{19}F$] FMISO was taken up in a greater amount by nitroimidazole derivative susceptible Mycobacterium tuberculosis in the resting stage of division than by nitroimidazole derivative non-susceptible E.coli under an anaerobic environment. Utilizing this new finding, for example, by labeling FMISO with a radionuclides according to the intended use, it becomes capable of detecting Mycobacterium tuberculosis in the resting stage of division by a non-invasive diagnostic method using positron emission tomography (PET), nuclear magnetic resonance device, or similar means, and thus can be used as a diagnostic imaging agent for determination of the therapeutic efficacy for Mycobacterium tuberculosis in the resting stage of division, a diagnostic imaging agent for latent tuberculosis, and the like.

## Claims

1. A diagnostic agent for infectious diseases caused by a nitroimidazole-susceptible microorganism, comprising an imidazole derivative or a fused imidazole derivative having at least one nitro group on an imidazole ring, or a labeled form thereof as an active ingredient.

2. The diagnostic agent according to Claim 1, wherein the imidazole derivative or the fused imidazole derivative is a compound represented by the following formula (1) :

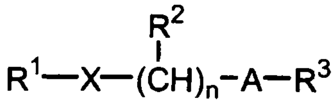

(1)

wherein, X represents an imidazole ring or a fused imidazole ring having at least one nitro group on an imidazole ring, $R^1$ represents a hydrogen atom or an alkyl group, n units of $R^2$ may be the same or different and each represent a hydrogen atom, a hydroxyl group, or an alkyl group, n represents a number of 0 to 5, A represents a single bond, an oxygen atom, a sulfur atom, $SO_2$, NH, CO, NHCO, or CONH, $R^3$ represents a hydrogen atom, an optionally substituted alkyl group, an optionally substituted aryl group, an optionally substituted aralkyl group, or an optionally substituted heterocyclic group.

3. The diagnostic agent according to Claim 1 or 2, wherein the active ingredient is a compound represented by the following formula (a-1), (b-1), or (c-1), or a labeled form thereof:

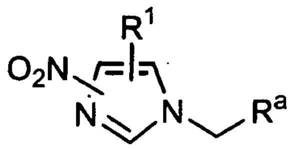

(a-1)

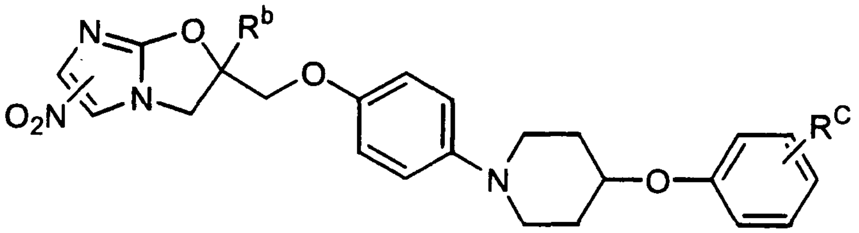

(b-1)

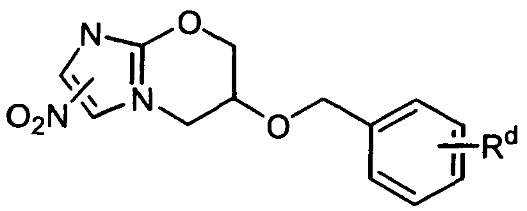

(c-1)

wherein, $R^1$ represents a hydrogen atom or a $C_{1-5}$ alkyl group, $R^a$ represents a $C_{1-5}$ alkyl group optionally having 1 to 3 substituents selected from a halogen atom, a hydroxyl group, and an alkanoyloxy group, a $C_{1-5}$ alkoxy group optionally having 1 to 3 substituents selected from a halogen atom, a hydroxyl group, and an alkanoyloxy group, or a benzoylaminomethyl group optionally having 1 to 3 substituents selected from a halogen atom, a hydroxyl group, and an alkanoyl group, $R^b$ represents a $C_{1-5}$ alkyl group, and $R^c$ and $R^d$ each represent a halogenoalkoxy group.

**4.** The diagnostic agent according to any one of Claims 1 to 3, wherein the active ingredient is metronidazole, tinidazole, benznidazole, fluoromisonidazole, ornidazole, nimorazole, 1-[2-(2-methyl-5-nitro-1H-1-imidazolyl)ethyl]-3-iodobenzamide, 1-[2-(2-nitro-1H-1-imidazolyl)ethyl]-3-iodobenzamide, (S)-2-nitro-6-((4-trifluoromethoxy)-benzyloxy)-6,7-dihydro-5H-imidazole[2,1-b][1,3] oxazine, and (R)-2-methyl-6-nitro-2-((4-(4-(4-(trifluoromethoxy)phenoxy)-piperidin-1-yl)phenoxy)methyl)-2,3-dihydroimidazo[2,1-b]oxazole, or a labeled form thereof.

**5.** The diagnostic agent according to any one of Claims 1 to 4, wherein the microorganism is obligate anaerobes, acid-fast bacteria, microaerophilic bacteria, or protozoa.

**6.** The diagnostic agent according to any one of Claims 1 to 5, wherein the microorganism is Mycobacterium tuberculosis, Helicobacter pylori, Trichomonas, Entamoeba histolytica, or Giardia lamblia.

**7.** An imidazole derivative or a fused imidazole derivative having at least one nitro group on an imidazole ring, or a labeled form thereof for use in the diagnosis of infectious diseases caused by nitroimidazole susceptible microorganisms.

**8.** The compound according to Claim 7, wherein the imidazole derivative or the fused imidazole derivative is a compound represented by the following formula (1) :

$$R^1-X-(\overset{R^2}{\overset{|}{C}}H)_n-A-R^3 \qquad (1)$$

wherein, X represents an imidazole ring or a fused imidazole ring having at least one nitro group on an imidazole ring, $R^1$ represents a hydrogen atom or an alkyl group, n units of $R^2$ may be the same or different and each represent a hydrogen atom, a hydroxyl group, or an alkyl group, n represents a number of 0 to 5, A represents a single bond, an oxygen atom, a sulfur atom, $SO_2$, NH, CO, NHCO, or CONH, $R^3$ represents a hydrogen atom, an optionally substituted alkyl group, an optionally substituted aryl group, an optionally substituted aralkyl group, or an optionally substituted heterocyclic group.

**9.** The compound according to Claim 7 or 8, wherein the compound is a compound represented by the following formula (a-1), (b-1), or (c-1), or a labeled form thereof:

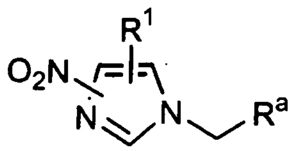

(a-1)

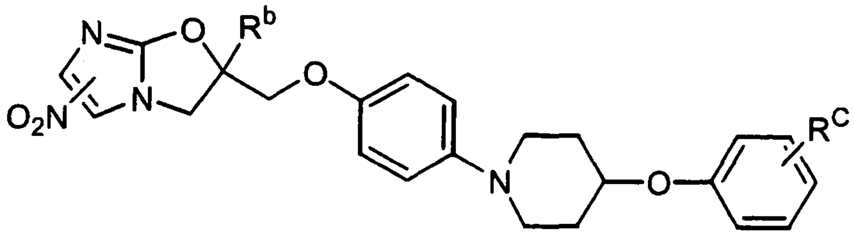

(b-1)

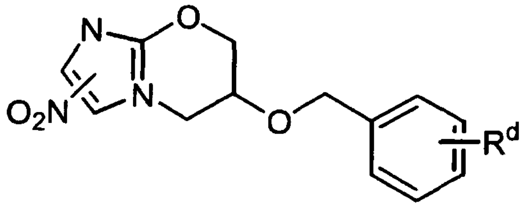

(c-1)

wherein, $R^1$ represents a hydrogen atom or a $C_{1-5}$ alkyl group, $R^a$ represents a $C_{1-5}$ alkyl group optionally having 1 to 3 substituents selected from a halogen atom, a hydroxyl group, and an alkanoyloxy group, a $C_{1-5}$ alkoxy group optionally having 1 to 3 substituents selected from a halogen atom, a hydroxyl group, and an alkanoyloxy group, or a benzoylaminomethyl group optionally having 1 to 3 substituents selected from a halogen atom, a hydroxyl group, and an alkanoyl group, $R^b$ represents a $C_{1-5}$ alkyl group, and $R^c$ and $R^d$ each represent a halogenoalkoxy group.

**10.** The compound according to any one of Claims 7 to 9, wherein the compound is metronidazole, tinidazole, benznidazole, fluoromisonidazole, ornidazole, nimorazole, 1-[2-(2-methyl-5-nitro-1H-1-imidazolyl)ethyl]-3-iodobenzamide, 1-[2-(2-nitro-1H-1-imidazolyl)ethyl]-3-iodobenzamide, (S)-2-nitro-6-((4-trifluoromethoxy)-benzyloxy)-6,7-dihydro-5H-imidazole[2,1-b][1,3] oxazine, and (R)-2-methyl-6-nitro-2-((4-(4-(4-(trifluoromethoxy)phenoxy)-piperidin-1-yl) phenoxy)methyl)-2,3-dihydroimidazo[2,1-b]oxazole, or a labeled form thereof.

**11.** The compound according to any one of Claims 7 to 10, wherein the microorganism is obligate anaerobes or acid-fast bacteria.

**12.** The compound according to any one of Claims 7 to 11, wherein the microorganism is Helicobacter pylori, Mycobacterium tuberculosis, or protozoa.

**13.** A diagnostic method for an infectious disease caused by a nitroimidazole susceptible microorganism, comprising using an imidazole derivative or a fused imidazole derivative having at least one nitro group on an imidazole ring, or a labeled form thereof.

**14.** The diagnostic method according to Claim 13, wherein the imidazole derivative or the fused imidazole derivative is a compound represented by the following formula (1) :

$$R^1-X-(\overset{R^2}{\overset{|}{C}}H)_n-A-R^3 \qquad (1)$$

wherein, X represents an imidazole ring or a fused imidazole ring having at least one nitro group on an imidazole ring, $R^1$ represents a hydrogen atom or an alkyl group, n units of $R^2$ may be the same or different and each represent a hydrogen atom, a hydroxyl group, or an alkyl group, n represents a number of 0 to 5, A represents a single bond, an oxygen atom, a sulfur atom, $SO_2$, NH, CO, NHCO, or CONH, $R^3$ represents a hydrogen atom, an optionally substituted alkyl group, an optionally substituted aryl group, an optionally substituted aralkyl group, or an optionally substituted heterocyclic group.

**15.** The diagnostic method according to Claim 13 or 14, wherein the active ingredient is a compound represented by the following formula (a-1), (b-1), or (c-1), or a labeled form thereof:

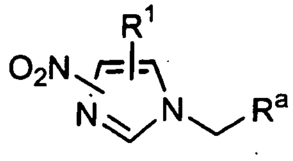

(a-1)

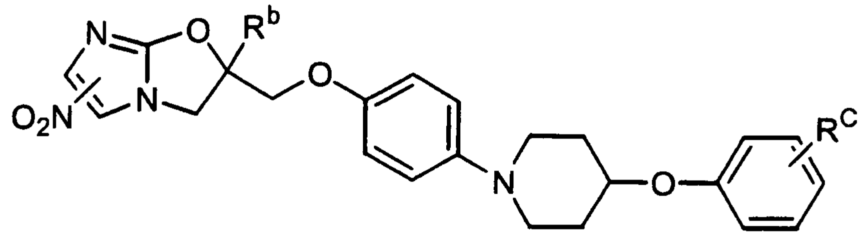

(b-1)

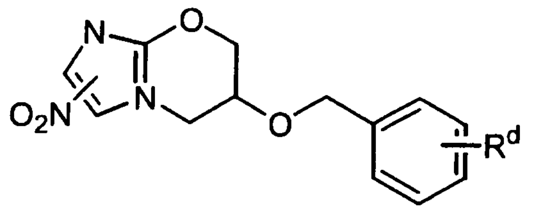

(c-1)

wherein, $R^1$ represents a hydrogen atom or a $C_{1-5}$ alkyl group, $R^a$ represents a $C_{1-5}$ alkyl group optionally having 1 to 3 substituents selected from a halogen atom, a hydroxyl group, and an alkanoyloxy group, a $C_{1-5}$ alkoxy group optionally having 1 to 3 substituents selected from a halogen atom, a hydroxyl group, and an alkanoyloxy group, or a benzoylaminomethyl group optionally having 1 to 3 substituents selected from a halogen atom, a hydroxyl group, and an alkanoyl group, $R^b$ represents a $C_{1-5}$ alkyl group, and $R^c$ and $R^d$ each represent a halogenoalkoxy group.

**16.** The diagnostic method according to any one of Claims 13 to 15, wherein the active ingredient is metronidazole, tinidazole, benznidazole, fluoromisonidazole, ornidazole, nimorazole, 1-[2-(2-methyl-5-nitro-1H-1-imidazolyl)ethyl]-3-iodobenzamide, 1-[2-(2-nitro-1H-1-imidazolyl)ethyl]-3-iodobenzamide, (S)-2-nitro-6-((4-trifluoromethoxy)-benzyloxy)-6,7-dihydro-5H-imidazole[2,1-b][1,3] oxazine, and (R)-2-methyl-6-nitro-2-((4-(4-(4-(trifluoromethoxy)phenoxy)-piperidin-1-yl)phenoxy)methyl)-2,3-dihydroimidazo[2,1-b]oxazole, or a labeled form thereof.

**17.** The diagnostic method according to any one of Claims 13 to 16, wherein the microorganism is obligate anaerobes or acid-fast bacteria.

**18.** The diagnostic method according to any one of Claims 13 to 17, wherein the microorganism is Helicobacter pylori, Mycobacterium tuberculosis, or protozoa.

**19.** A screening method for a therapeutic agent for an infectious disease caused by a microorganism, comprising detecting binding of an imidazole derivative or a fused imidazole derivative having at least one nitro group on an imidazole ring, or a labeled form of the derivative to a viable microorganism in the presence of a test substance.

Fig.1

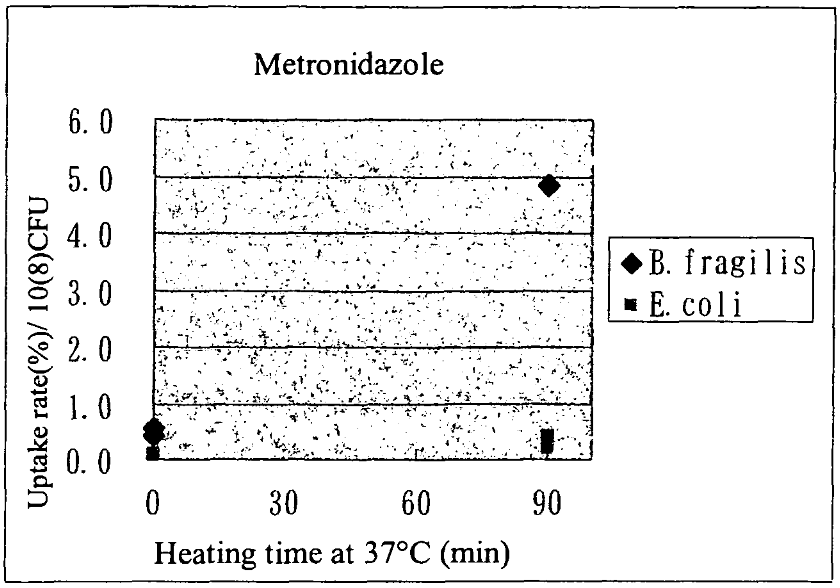
Metronidazole
6. 0
5. 0
4. 0
3. 0
2. 0
1. 0
0. 0
Uptake rate(%)/ 10(8)CFU
0
30
60
90
Heating time at 37°C (min)
B. fragilis
E. coli

Fig.2

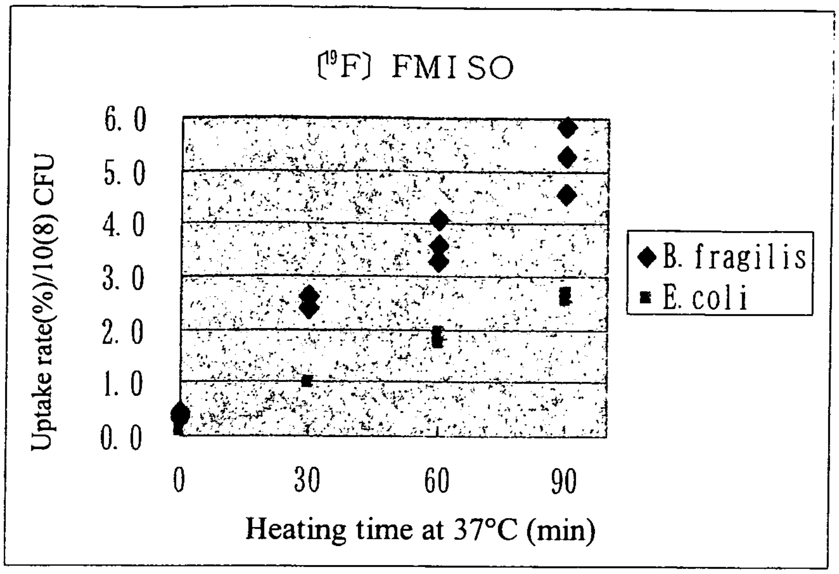
[19F] FMISO
6.0
5.0
4.0
3.0
2.0
1.0
0.0
Uptake rate(%)/10(8) CFU
0
30
60
90
Heating time at 37°C (min)
B. fragilis
E. coli

Fig.3

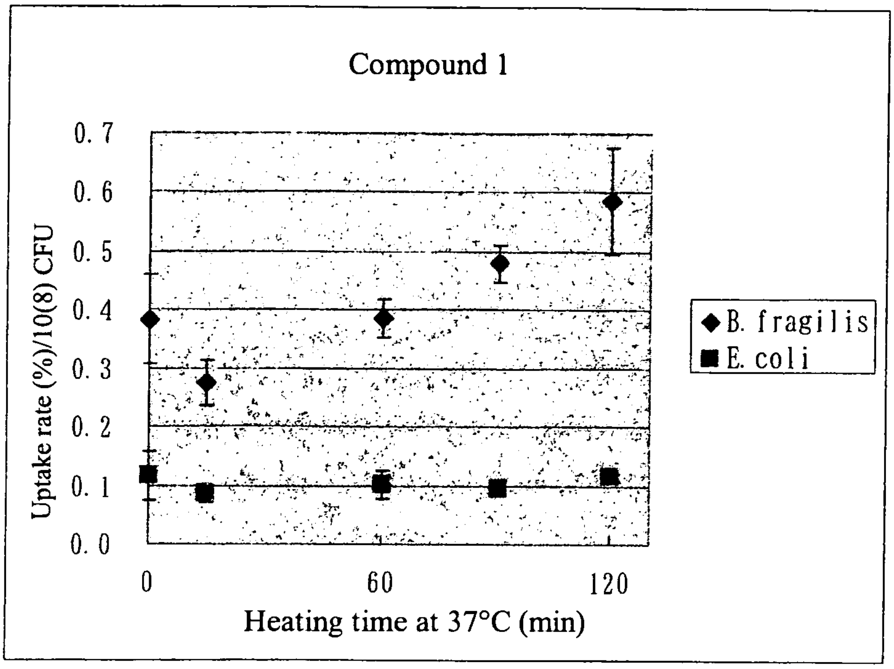
Compound 1
Uptake rate (%)/10(8) CFU
0. 7
0. 6
0. 5
0. 4
0. 3
0. 2
0. 1
0. 0
0
60
120
Heating time at 37°C (min)
B. fragilis
E. coli

Fig.4

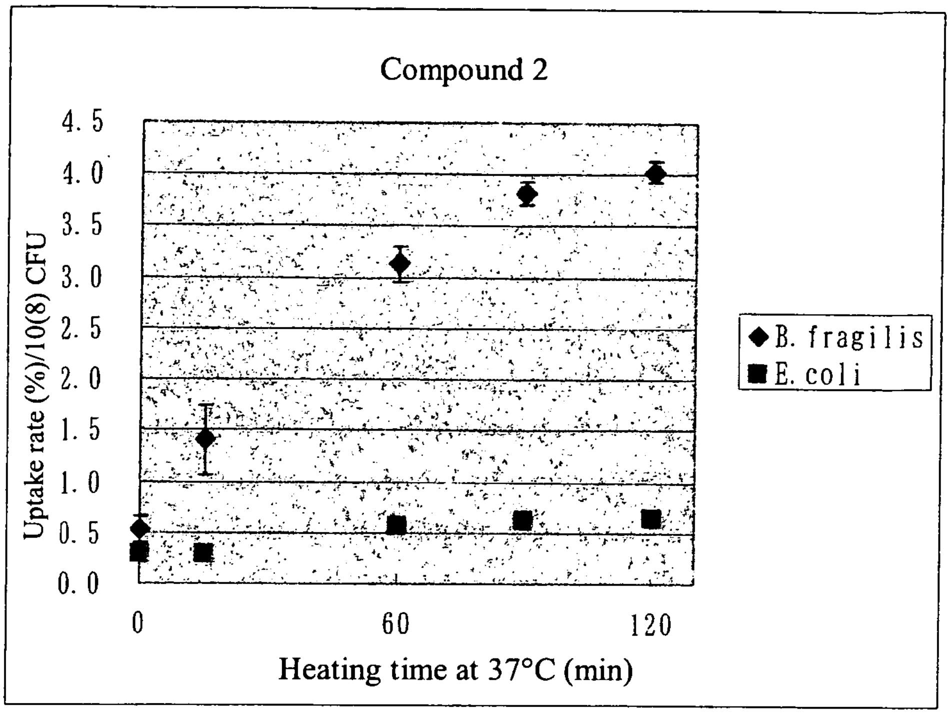
Compound 2
Uptake rate (%)/10(8) CFU
4.5
4.0
3.5
3.0
2.5
2.0
1.5
1.0
0.5
0.0
0
60
120
Heating time at 37°C (min)
B. fragilis
E. coli

Fig.5

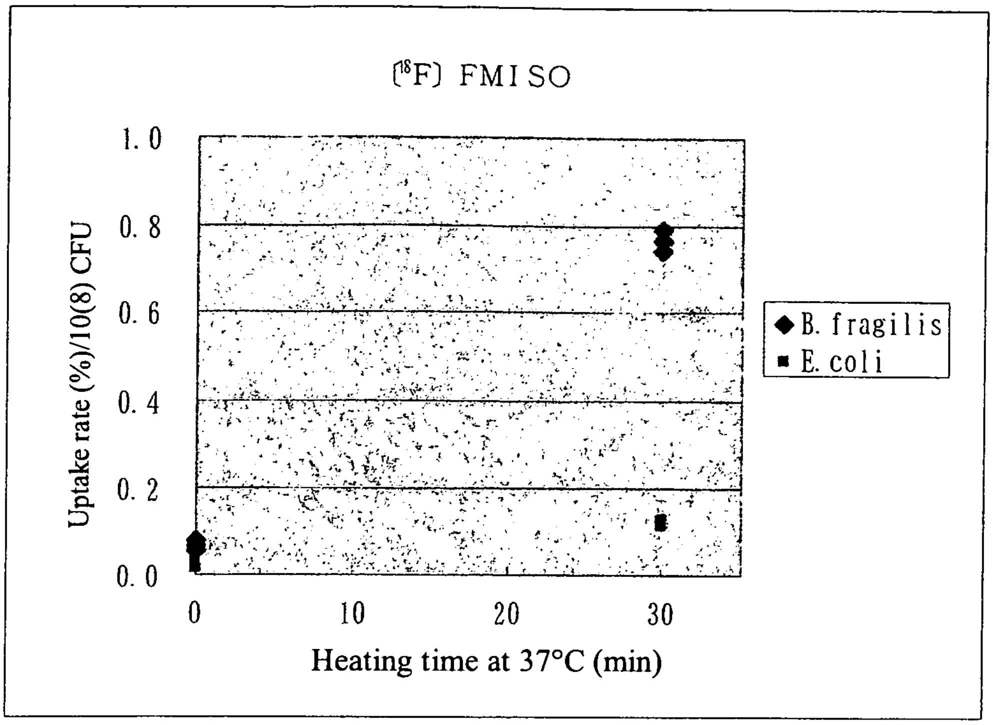
[18F] FMISO
1.0
0.8
0.6
0.4
0.2
0.0
Uptake rate (%)/10(8) CFU
0
10
20
30
Heating time at 37°C (min)
B. fragilis
E. coli

Fig.6

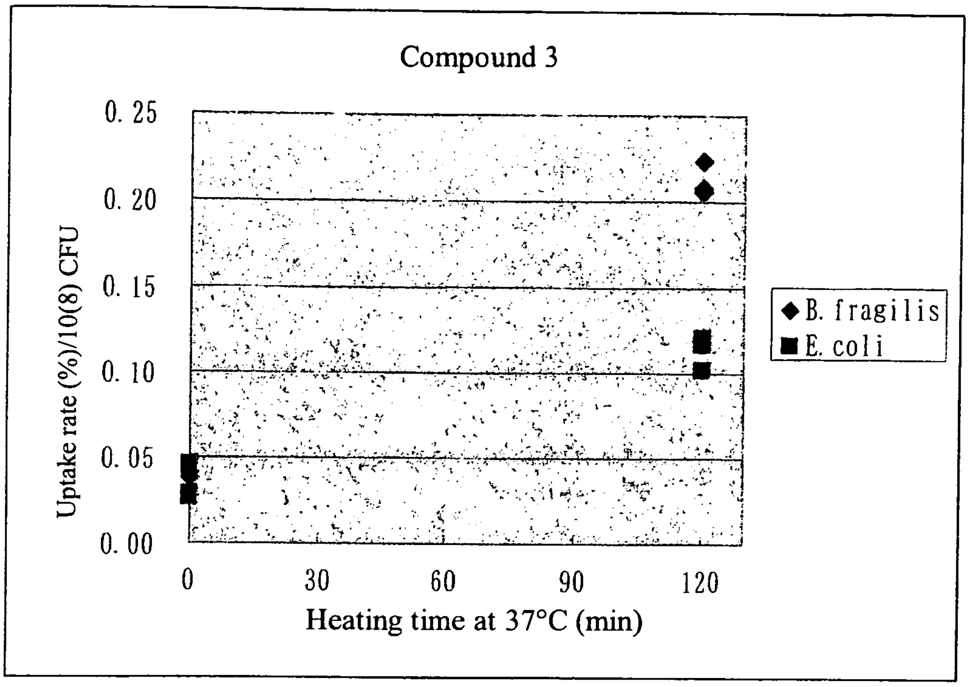
Compound 3
0.25
0.20
0.15
0.10
0.05
0.00
Uptake rate (%)/10(8) CFU
0
30
60
90
120
Heating time at 37°C (min)
B. fragilis
E. coli

Fig.7

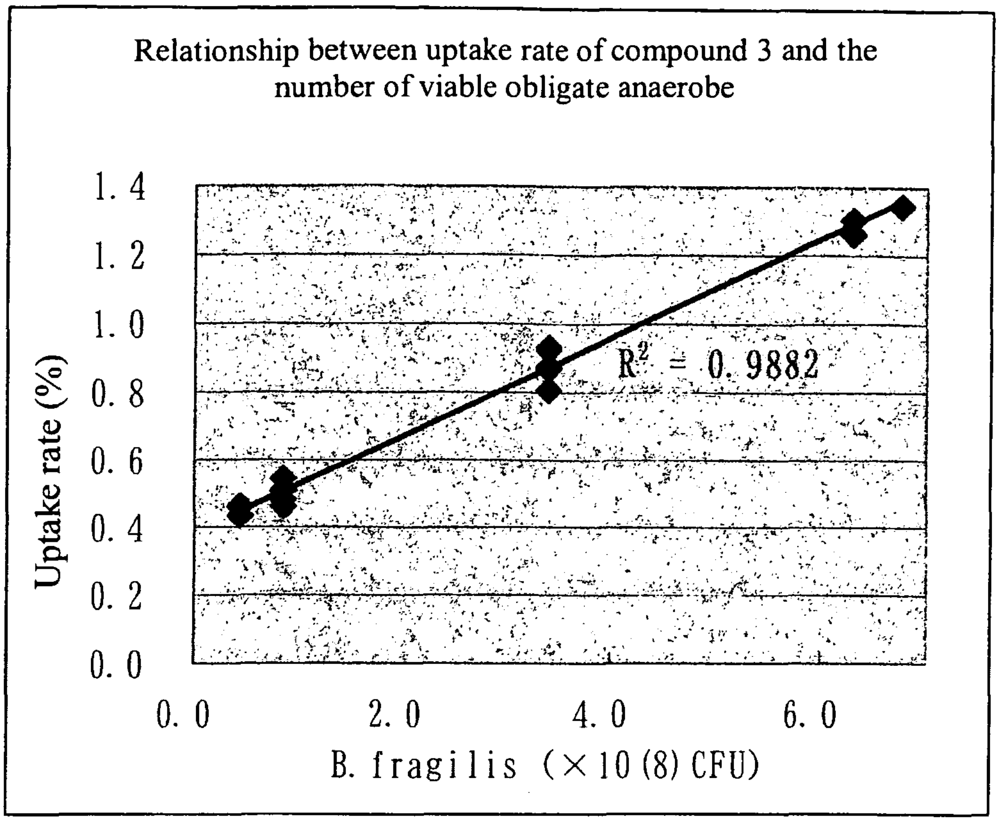
Relationship between uptake rate of compound 3 and the number of viable obligate anaerobe
Uptake rate (%)
1.4
1.2
1.0
0.8
0.6
0.4
0.2
0.0
0.0
2.0
4.0
6.0
B. fragilis (×10(8) CFU)
R2 = 0.9882

Fig.8

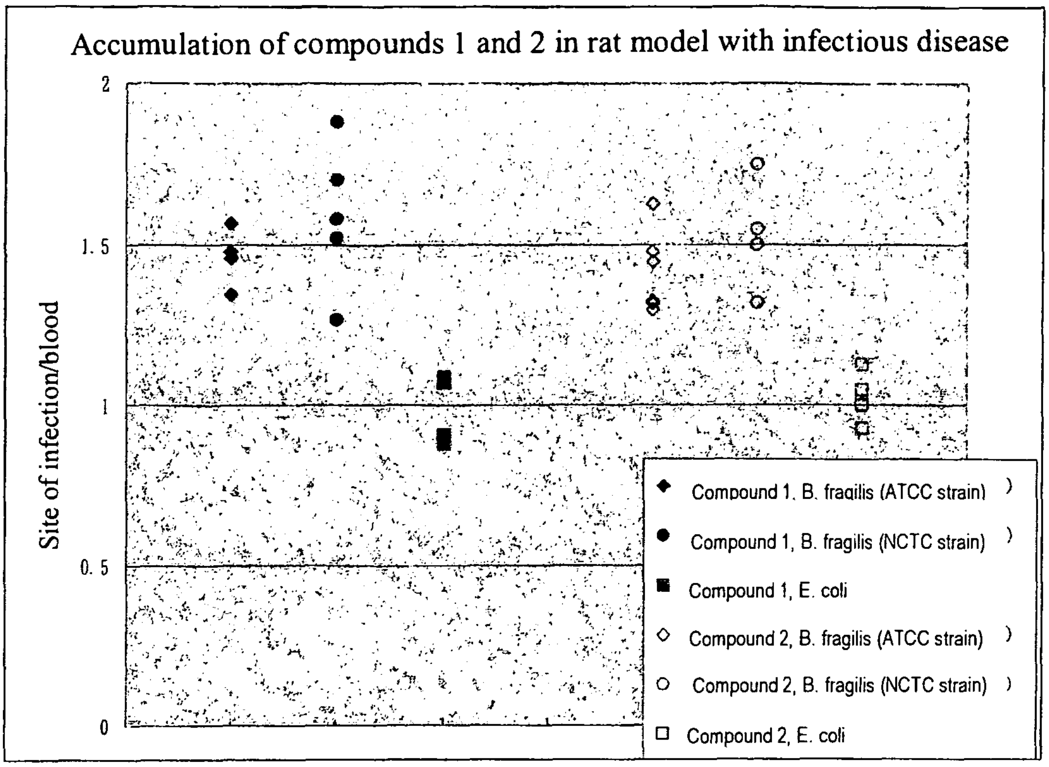
Accumulation of compounds 1 and 2 in rat model with infectious disease
Site of infection/blood
2
1.5
1
0.5
0
Compound 1. B. fragilis (ATCC strain)
Compound 1, B. fragilis (NCTC strain)
Compound 1, E. coli
Compound 2, B. fragilis (ATCC strain)
Compound 2, B. fragilis (NCTC strain)
Compound 2, E. coli

Fig.9

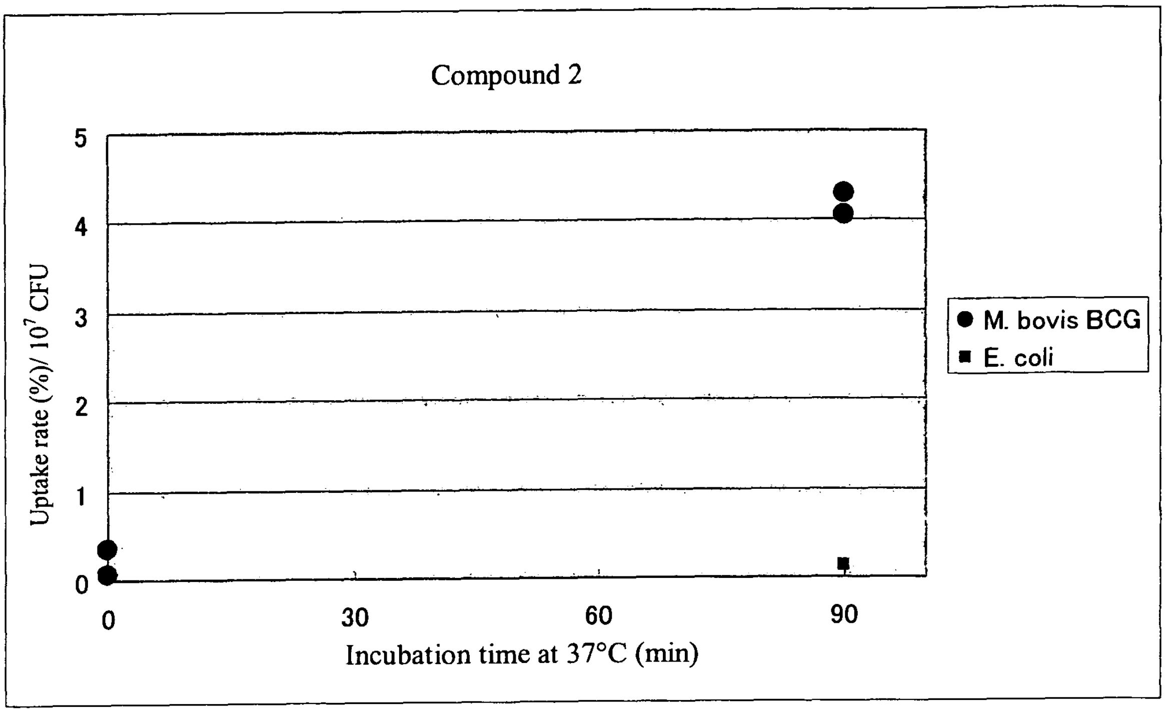
Compound 2
Uptake rate (%)/ 10^7 CFU
5
4
3
2
1
0
0
30
60
90
Incubation time at 37°C (min)
M. bovis BCG
E. coli

Fig.10

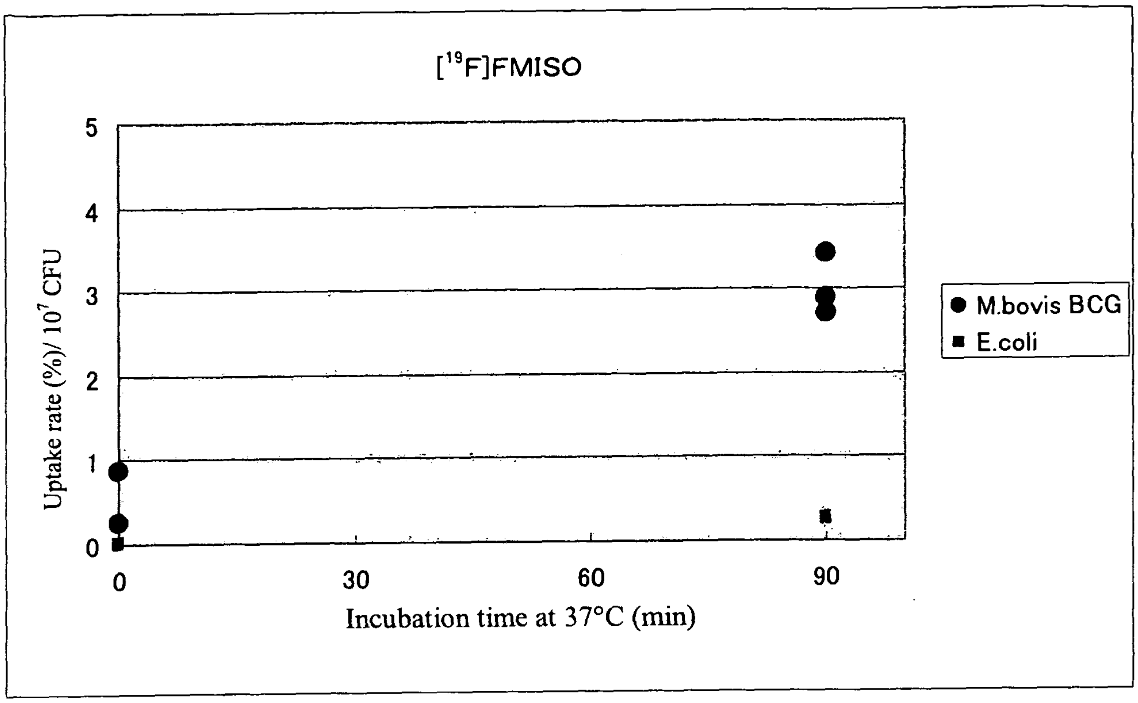
[19F]FMISO
Uptake rate (%)/ 10^7 CFU
5
4
3
2
1
0
0
30
60
90
Incubation time at 37°C (min)
M.bovis BCG
E.coli

**INTERNATIONAL SEARCH REPORT**

International application No.
PCT/JP2010/068812

A. CLASSIFICATION OF SUBJECT MATTER
*G01N33/50*(2006.01)i, *A61K49/00*(2006.01)i, *G01N33/15*(2006.01)i

According to International Patent Classification (IPC) or to both national classification and IPC

B. FIELDS SEARCHED

Minimum documentation searched (classification system followed by classification symbols)
G01N33/50, A61K49/00, G01N33/15

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| Y | JP 2003-509413 A (Yoshihiro TAKAI), 11 March 2003 (11.03.2003), entire text<br>& US 6743925 B1 & EP 1218351 A<br>& WO 2001/019799 A2 & DE 60019622 D<br>& DE 60019622 T & AU 6878400 A<br>& CA 2384879 A & AT 293607 T<br>& DK 1218351 T & ES 2237447 T | 1-19 |
| Y | JP 2008-505936 A (The University of Sydney), 28 February 2008 (28.02.2008), paragraphs [0070] to [0071]<br>& US 2009/0092550 A1 & EP 1773819 A<br>& WO 2006/005137 A1 & CA 2573927 A | 1-19 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 11 November, 2010 (11.11.10) | 22 November, 2010 (22.11.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/068812

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-507354 A (Universite Catholique de Louvain), 25 February 2003 (25.02.2003), paragraph [0008] & US 7108846 B1 & EP 1202945 A & WO 2001/012575 A1 & DE 60022058 D & DE 60022058 T & AU 5215100 A & CA 2378712 A & AT 302175 T & ES 2246858 T | 1-19 |
| A | Martin H. Cherk et al., Lack of Correlation of Hypoxic Cell Fraction and Angiogenesis with Glucose Metabolic Rate in Non-Small Cell Lung Cancer Assessed by 18F-Fluoromisonidazole and 18F-FDG PET, J Nucl Med, 2006, 47, 1921-1926 | 1-19 |
| A | TEWSON T J, NUCLEAR MEDICINE AND BIOLOGY, 1997.11.01, V24 N8, P755-760 | 1-19 |

Form PCT/ISA/210 (continuation of second sheet) (July 2009)

**INTERNATIONAL SEARCH REPORT**

International application No.
PCT/JP2010/068812

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The inventions in claims 1 - 18 relate to diagnostic agents for infective diseases; a labeled body for use in diagnosis of infective diseases; and a method for diagnosis of infective diseases.

The invention in claim 19 relates to a method for screening therapeutic drugs for infective diseases.

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003509413 A
- JP 2009521464 A
- JP 57181066 A
- JP 4056031 B
- JP 11508270 A
- JP 9143071 A
- JP 2005330266 A
- JP 2004149527 A

### Non-patent literature cited in the description

- Kokinyaku shiyo no guideline. *The Japanese Association for Infectious Diseases, Japanese Society of Chemotherapy*
- Kenkiseikin kansensho shindan/chiryo guideline 2007. *Japanese Society of Chemotherapy, Nihon kenkiseikin kansennsho kenkyukai*
- **SALONEN JH.** *Clinical Infectious Diseases,* 1998, vol. 26, 1413-1417
- **CHU T.** *Bioorg Med Chem Lett. 9,* 2004, vol. 14 (3), 747-749
- **MUKAI T.** *Bioorg Med Chem. 1,* 2009, vol. 17 (13), 4285-4289
- **CHERK MH.** *J Nucl Med.,* 2006, vol. 47 (12), 1921-1926
- **LIU RS.** *Eur J Nucl Med.,* 1996, vol. 23 (10), 1384-1387
- **SAMUELSON J.** *Antimicrob Agents Chemother,* 1999, vol. 43 (7), 1533-1541
- **PRINCE HN.** *Appl Microbiol.,* 1969, vol. 18 (5), 728-730
- The Merck Manuals
- Cowan and Steel's Manual for the Identification of Medical Bacteria. 29
- **SUN Z.** *Tuber Lung Dis.,* 1999, vol. 79 (5), 319-320
- **SINGH R.** *Science,* 2008, vol. 322 (5906), 1392-1395
- **KIM P.** *J Med Chem.,* 2009, vol. 52, 1317-1328